# EUROPEAN PATENT SPECIFICATION

(11) **EP 1 742 608 B1**
(45) Date of publication and mention of the grant of the patent: **24.02.2010**
(21) Application number: 05743226.2
(22) Date of filing: 29.04.2005
(51) Int. Cl.: A61K 8/40, A61Q 5/00, A61Q 19/10, C07C 275/42

(54) **BIAROMATIC COMPOUNDS WHICH ACTIVATE PPAR(GAMMA) TYPE RECEPTORS, THEIR PROCESS OF PREPARATION AND THEIR USE IN COSMETIC OR PHARMACEUTICAL COMPOSITIONS**
BIAROMATISCHE VERBINDUNGEN, DIE REZEPTOREN DES TYPS PPAR(GAMMA) AKTIVIEREN, VERFAHREN ZU DEREN HERSTELLUNG UND DEREN VERWENDUNG IN KOSMETISCHEN ODER PHARMAZEUTISCHEN ZUSAMMENSETZUNGEN
COMPOSES BIAROMATIQUES ACTIVANT DES RECEPTEURS DE TYPE PPAR(GAMMA), LEUR PROCESSUS DE PREPARATION ET LEUR UTILISATION DANS DES COMPOSITIONS COSMETIQUES OU PHARMACEUTIQUES

(30) Priority: 06.05.2004 FR 0404913; 26.05.2004 US 574217 P
(43) Date of publication of application: 17.01.2007
(73) Proprietor: Galderma Research & Development, 06410 Biot (FR)
(72) Inventor: AUBERT, Jérôme, St Jacques, FR-06130 Grasse (FR); CLARY, Laurence, FR-06480 La Colle sur Loup (FR); MAUVAIS, Pascale, FR-06600 Antibes (FR); RIVIER, Michel, FR-06100 Nice (FR); THOREAU, Etienne, F-06460 Saint Vallier de Thiey (FR); BOITEAU, Jean-Guy, FR-34130 Saint Aunes (FR)
(74) Representative: Allab, Myriam
(86) International application number: PCT/EP2005/005797
(87) International publication number: WO 2005/108352

(56) References cited:
- WO-A-02/12210
- WO-A-2004/048351

## Description

The invention relates, as novel and useful industrial products, to a novel class of biaromatic compounds which are activators of receptors of "Peroxisome Proliferator-Activated Receptor" type of subtype γ (PPARγ). It also relates to their process of preparation and to their use in pharmaceutical compositions intended for use in human or veterinary medicine or alternatively in cosmetic compositions.

The activity of receptors of PPAR type has formed the subject of many studies. Mention may be made, by way of indication, of the publication entitled "Differential Expression of Peroxisome Proliferator-Activated Receptor Subtypes During the Differentiation of Human Keratinocytes", Michel Rivier et al., J. Invest. Dermatol., 1998, 111, 1116-1121, in which a large number of bibliographic references relating to receptors of PPAR type are listed. Mention may also be made, by way of indication, of the report entitled "The PPARs: From Orphan Receptors to Drug Discovery", Timothy M. Willson et al., J. Med. Chem., 2000, 43, 527-550.

PPAR receptors activate transcription by binding to elements of DNA sequences, known as peroxisome proliferator response elements (PPRE), in the form of a heterodimer with retinoid X receptors (known as RXRs).

Three subtypes of human PPARs have been identified and described: PPARα, PPARγ and PPARδ (or NUC1).

PPARα is mainly expressed in the liver, while PPARδ is ubiquitous.

PPARγ is the most widely studied of the three subtypes. All the references suggest a critical role for PPARγ in the regulation of the differentiation of adipocytes, where it is strongly expressed. It also plays a key role in systemic lipid homeostasis.

Furthermore, the Applicant Company has already disclosed, in Patent Application FR98/02894, the use of PPARγ-activating compounds in the preparation of a pharmaceutical composition, the composition being intended for the treatment of skin disorders related to an anomaly in the differentiation of the epidermal cells.

The Applicant Company has also disclosed a class of biaromatic compounds which are activators of PPARγ receptors in its Patent FR 2 812 876.

One of the aims of the present invention is to provide a novel class of PPARγ-activating compounds exhibiting biological activities which are significantly improved with respect to those of the compounds known in the state of the art and in particular with respect to those disclosed in Patent FR 2 812 876.

According to the present invention, the Applicant Company has discovered a restricted group of compounds, corresponding to the formula (I) set out below, which exhibit a surprising biological activity, in particular a binding affinity for PPARγ receptors which is significantly increased with respect to that of the compounds of Patent FR 2 812 876. This increased binding affinity emerges in particular from apparent dissociation constant (KdApp) values which are surprisingly lowered, as explained below.

Thus, the present invention relates to compounds corresponding to the following general formula (I): 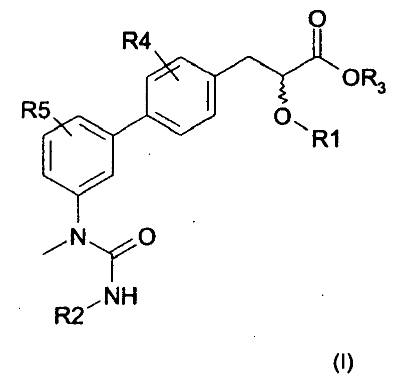 in which:
- R1 represents an alkyl group having 1 to 6 carbon atoms chosen from the methyl, ethyl, n-propyl, isopropyl, isobutyl, tert-butyl, n-pentyl, isopentyl, n-hexyl, and isohexyl radicals, a cyclopropyl, a cyclohexyl, an ethylenyl, an allyl, a propenyl, a butenyl, a pentenyl or an hexenyl radical, an acetyl group, a methylcyclopropane group, an aralkyl group or an aryl group, the aryl group being chosen from a biphenyl, cinnamyl or naphthyl radical which can be mono- or disubstituted by a halogen atom, a CF₃ radical, an alkyl radical having from 1 to 6 carbon atoms, an alkoxy radical having from 1 to 6 carbon atoms, a nitro functional group, a polyether radical, an aryl radical, a benzoyl radical, an alkyl ester group, a carboxylic acid, a hydroxyl radical optionally protected by an acetyl or benzoyl group, or an amino functional group optionally protected by an acetyl-or benzoyl group or optionally substituted by at least one alkyl having from 1 to 6 carbon atoms, and the aralkyl group being chosen from a phenethyl or naphth-2-ylmethyl radical which is unsubstituted or substituted by one or more radicals chosen from a halogen atom, a CF₃ radical, an alkyl radical having from 1 to 6 carbon atoms, an alkoxy radical having from 1 to 6 carbon atoms, a hydroxyl radical or an amino functional group which is unprotected or unsubstituted or optionally substituted by at least one alkyl having from 1 to 6 carbon atoms, or a carboxyl functional group;
- R2 represents an alkyl group having 3 to 8 carbon atoms or a cyclopropyl, a cyclopentyl, a cyclohexyl, an allyl, a propenyl, a butenyl, a pentenyl, an hexenyl, an heptenyl or an octenyl radical;
- R3 is a hydrogen atom;
- R4 and R5 represent a hydrogen atom;
and the possible isomers, optical and/or geometrical, pure or as a mixture, in all proportions, of the said compounds of formula (I) and the possible tautomeric forms, and also the salts of the said compounds of formula (I).

For the compounds of formula (I) which is presented above, the term "geometrical isomer" means *cis*/*trans* or E/Z isomerism. More particularly, the possible double bond or bonds present in the various substituents of the compounds of general formula (I) can be of E or Z configuration. These geometrical isomers, pure or impure, alone or as a mixture, form an integral part of the compounds of formula (I).

The term "optical isomer" embraces all the forms of isomers, alone or as a mixture, the presence of which results from one or more axes and/or centres of symmetry in the molecule which results in the rotation of a beam of polarized light. The term "optical isomer" comprises more particularly the enantiomers and the diastereoisomers, in the pure form or as a mixture.

When the compounds according to the invention are provided in the form of a salt, it is preferably an alkali metal salt, in particular the sodium salt, or an alkaline earth metal salt or an organic amine salt, more particularly of amino acids, such as arginine or lysine.

According to the present invention, the term "alkyl radical having from 1 to 6 carbon atoms" is understood to mean, preferably, an optionally branched, saturated, linear alkyl radical selected from the methyl, ethyl, n-propyl, isopropyl, n-butyl, isobutyl, tert-butyl, n-pentyl, isopentyl, n-hexyl, and isohexyl, radicals.

According to the present invention, the term "alkyl radical having from 3 to 8 carbon atoms" is understood to mean, preferably, an optionally branched, saturated, linear alkyl radical comprising 3 to 8 carbon atoms and preferably the n-propyl, isopropyl, n-butyl, isobutyl, tert-butyl, n-pentyl, isopentyl, n-hexyl, isohexyl, n-heptyl, isoheptyl, n-octyl, and isooctyl radicals.

The term "halogen atom" is understood to mean, preferably, a fluorine, chlorine or bromine atom.

The term "aralkyl radical" is understood to mean, preferably, a benzyl, phenethyl or naphth-2-yl-methyl radical which is unsubstituted or substituted by one or more radicals chosen from a halogen atom, a CF₃ radical, an alkyl radical having from 1 to 6 carbon atoms, an alkoxy radical having from 1 to 6 carbon atoms, a hydroxyl radical or an amino functional group which is unprotected or unsubstituted or optionally substituted by at least one alkyl having from 1 to 6 carbon atoms, or a carboxyl functional group.

The term "aryl radical" is understood to mean, preferably, a phenyl, biphenyl, cinnamyl or naphthyl radical which can be mono- or disubstituted by a halogen atom, a CF₃ radical, an alkyl radical having from 1 to 6 carbon atoms, an alkoxy radical having from 1 to 6 carbon atoms, a nitro functional group, a polyether radical, an aryl radical, a benzoyl radical, an alkyl ester group, a carboxylic acid, a hydroxyl radical optionally protected by an acetyl or benzoyl group, or an amino functional group optionally protected by an acetyl or benzoyl group or optionally substituted by at least one alkyl having from 1 to 6 carbon atoms.

The term "alkoxy radical" is understood to mean, preferably, a methoxy, ethoxy, isopropyloxy, tert-butoxy, hexyloxy, benzyloxy or phenoxy radical which can optionally be substituted by an alkyl radical having from 1 to 6 carbon atoms.

The term "polyether radical" is understood to mean, preferably, a radical having from 1 to 6 carbon atoms which is interrupted by at least one oxygen atom, such as the methoxymethoxy, methoxymethylene, ethoxymethoxy, ethoxymethylene or methoxyethoxymethoxy radicals.

The term "alkyl ester radical" is understood to mean a carboxylate functional group substituted by an alkyl radical having from 1 to 6 carbon atoms.

Mention may in particular be made, among the compounds of formula (I) above coming within the scope of the present invention, of the following compounds (alone or as a mixture):
1. 2(S)-Ethoxy-3-[3'-(3-heptyl-1-methylureido)-biphenyl-4-yl]propanoic acid,
2. 2(S)-Ethoxy-3-[3'-(1-methyl-3-pentylureido)-biphenyl-4-yl]propanoic acid,
3. 2(S)-Cyclopropylmethoxy-3-[3'-(3-heptyl-1-methylureido)biphenyl-4-yl]propanoic acid,
4. 2(S)-Propyloxy-3-[3'-(3-heptyl-1-methylureido)-biphenyl-4-yl]propanoic acid,
6. 2(S)-Allyloxy-3-[3'-(3-heptyl-1-methylureido)-biphenyl-4-yl]propanoic acid,
7. 2(S)-Cyclopropylmethoxy-3-[3'-(1-methyl-3-pentylureido)biphenyl-4-yl]propanoic acid,
8. 3-[3'-(1-Methyl-3-pentylureido)biphenyl-4-yl]-2(S)-propoxypropanoic acid,
9. 2(S)-Allyloxy-3-[3'-(1-methyl-3-pentylureido)-biphenyl-4-yl]propanoic acid,
11. 2(S)-Methoxy-3-[3'-(1-methyl-3-pentylureido)-biphenyl-4-yl]propanoic acid,
12. 3-[3'-(3-Heptyl-1-methylureido)biphenyl-4-yl]-2(S)-methoxypropanoic acid,
13. 2(S)-Ethoxy-3-[3'-(1-methyl-3-propylureido)-biphenyl-4-yl]propanoic acid,
14. 3-[3'-(3-Cyclopropylmethyl-1-methylureido)-biphenyl-4-yl]-2(S)-ethoxypropanoic acid,
15. 3-[3'-(3-Cyclopentylmethyl-1-methylureido)-biphenyl-4-yl]-2(S)-ethoxypropanoic acid,
21. 2(S)-Cyclopropylmethoxy-3-[3'-(3-cyclopropylmethyl-1-methylureido)biphenyl-4-yl]propanoic acid,
22. 3-{3'-[3-(2-Cyclohexylethyl)-1-methylureido]-biphenyl-4-yl}-2(S)-ethoxypropanoic acid,
23. 2(R)-Ethoxy-3-[3'-(1-methyl-3-pentylureido)-biphenyl-4-yl]propanoic acid,
24. 2(R)-Ethoxy-3-[3'-(3-heptyl-1-methylureido)biphenyl-4-yl]propanoic acid,
25. 2-Ethoxy-3-[3'-(3-heptyl-1-methylureido)biphenyl-4-yl]propanoic acid,
26. 2(R)-Allyloxy-3[3'-(3-heptyl-1-methylureido)-biphenyl-4-yl]propanoic acid,
27. 3-[3'-(3-Allyl-1-methylureido)biphenyl-4-yl]-2(S)-ethoxypropanoic acid.

According to the present invention, the compounds of formula (I) which are more particularly preferred are those which exhibit at least one of the following characteristics:
- R1 is chosen from an alkyl radical chosen from the methyl, ethyl, n-propyl, isopropyl, isobutyl or tert-butyl radicals, a cyclopropyl or a methylcyclopropane group,
- R2 represents an alkyl radical chosen from the n-pentyl, isopentyl, n-hexyl, isohexyl, n-heptyl or isoheptyl radicals, or a cyclopentyl or cyclohexyl radical,
- R3 represents a hydrogen atom,
- R4 and/or R5 are a hydrogen atom.

In particular, according to the present invention, preference will be given to the compounds of formula (I) exhibiting all the following characteristics:
- R1 is chosen from an alkyl radical chosen from the methyl, ethyl, n-propyl, isopropyl, isobutyl or tert-butyl radicals, a cyclopropyl a methylcyclopropane group,
- R2 represents an alkyl radical chosen from the n-pentyl, isopentyl, n-hexyl, isohexyl, n-heptyl or isoheptyl radicals, or a cyclopentyl or cyclohexyl-radical,
- R3 represents a hydrogen atom,
- R4 and/or R5 are a hydrogen atom.

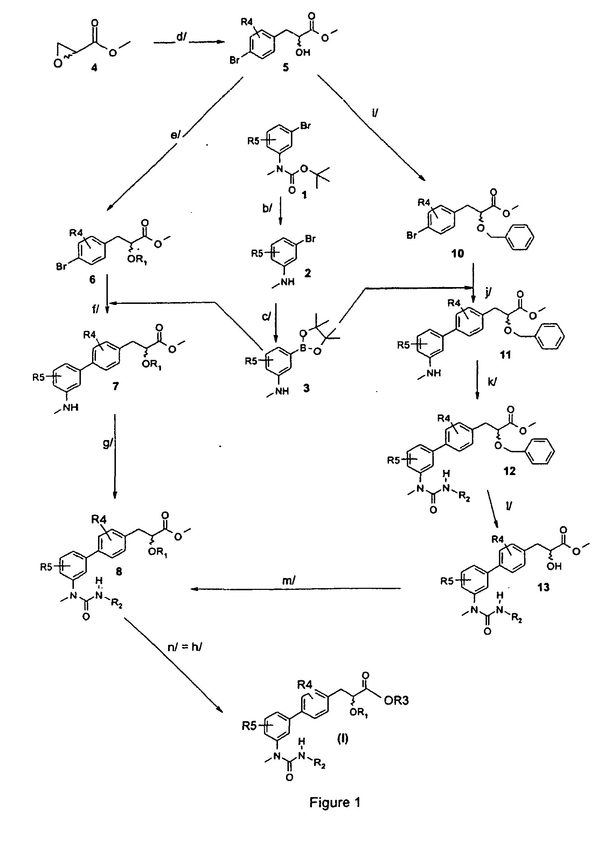

More particularly, the present invention relates to a process for the synthesis of the compounds corresponding to the general formula (I) or of the possible isomers, optical and/or geometrical, pure or as a mixture, in all proportions, of the said compounds of formula (I), of the possible tautomeric forms, or of the salts of the said compounds of formula (I), characterized in that it comprises the following stages:
in the stages described below, which are read with regard to **Figure 1**, unless otherwise indicated, the R1, R2, R3, R4 and R5 radicals of the compounds **1** to **20** are the same as those defined for the compounds of general formula (I).

a) Preparation of the compound of formula **1:** 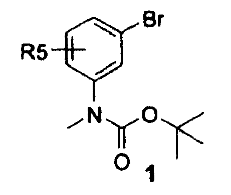 from commercial 3-bromoaniline optionally substituted by an R5 group, by protecting the amine with di(tert-butyl) dicarbonate and by then carrying out a methylation, for example with methyl iodide, in the presence of sodium hydride;
b) Preparation of compound **2**: 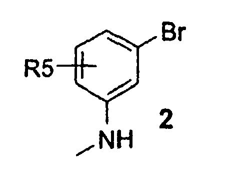 by treating compound **1** with an acid, such as, for example, trifluoroacetic acid;
c) Preparation of compound **3**: 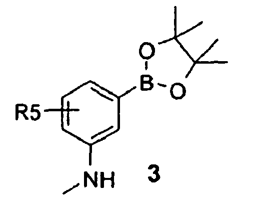 by the reaction of compound **2** with pinacolborane in the presence of a catalyst, such as palladium dichloride diphenylphosphinopropane ferrocene;
d) Preparation of compound **5:** 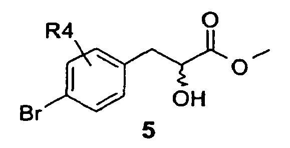 by treating the commercial epoxide **4:** 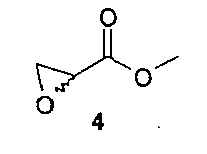 with an aryl cuprate obtained by reaction of an aryl halide, such as, for example, 1,4-dibromobenzene, in the presence of tert-butyllithium and of copper cyanide;
e) Preparation of compound **6:** 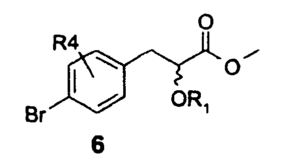 by reacting compound **5** with an alkyl halide, such as ethyl iodide, for example, in the presence of silver oxide, to prevent any problem of racemization;
f) Preparation of compound **7:** 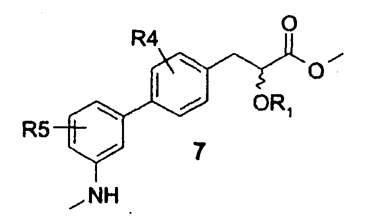 by the coupling of compound **6** and compound **3** according to a reaction of Suzuki type in the presence of tetrakis(triphenylphosphine)palladium;
g) Preparation of compound **8:** 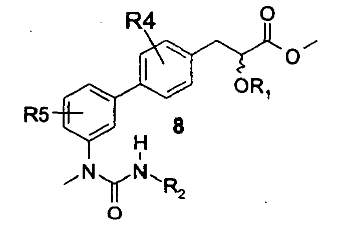 by the reaction between compound **7** and an alkyl isocyanate, such as, for example, heptyl isocyanate;
h) Preparation of **compound (I):** 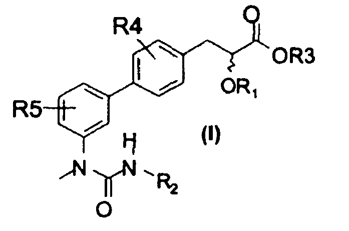
when R3 is a hydrogen atom: by saponification of compound **8** in the presence of a base, such as, for example, sodium hydroxide.

According to another advantageous process for the synthesis of the compounds of formula (I), the stages for preparing compound **3** are repeated as defined in parts a) to c) and the stages for preparing compound **5** are repeated as defined in part d) and are followed by the stages set out below:
i) Preparation of compound **10:** 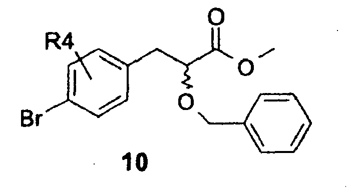 by treating compound **5** with a benzyl halide, such as, for example, benzyl bromide, in the presence of silver oxide;
j) Preparation of compound **11:** 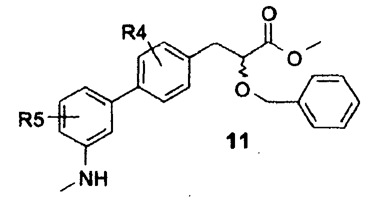 by coupling compound **10** with compound **3** by a reaction of Suzuki type using a palladium catalyst, such as, for example, tetrakis(triphenylphosphine)palladium;
k) Preparation of compound **12:** 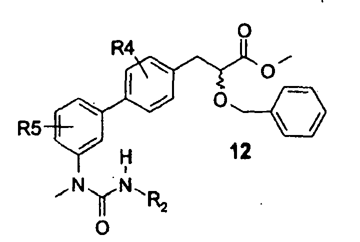 by the reaction between compound **11** and an alkyl isocyanate, such as, for example, heptyl isocyanate;
l) Preparation of the alcohol **13:** 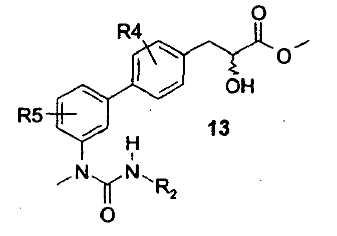 by hydrogenolysis of compound **12** with hydrogen in the presence of palladium-on-charcoal;
m) Preparation of compound **8:** 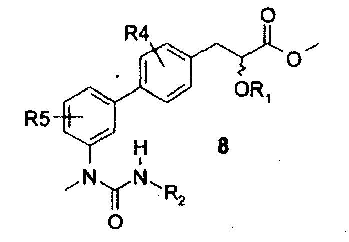 by reacting compound **13** with an alkyl halide, such as allyl bromide, for example, in the presence of silver oxide;
n) Preparation of compound **(I)** from compound **8** as defined in stage h).

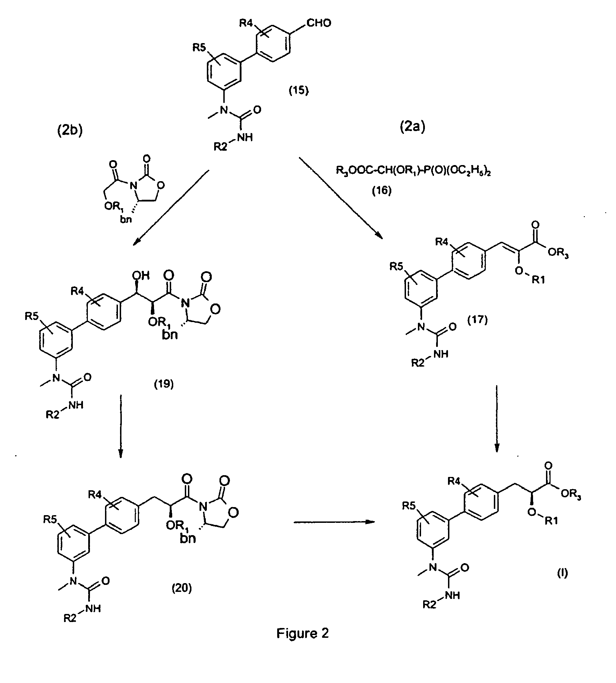

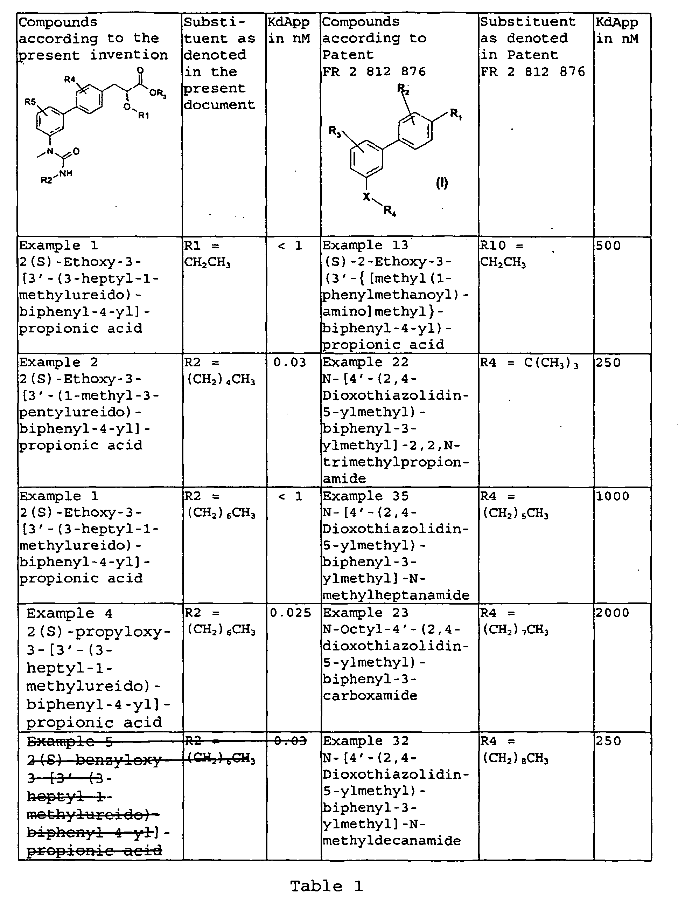

The compounds of formula (I) can also be obtained according to the reaction scheme presented by the synthetic route **2a** of **Figure 2** from aldehyde derivatives **15** according to a reaction of Horner type with a phosphonate **16** in the presence of a base, such as sodium hydride, butyllithium or potassium tert-butoxide, then hydrogenation in the presence of palladium-on-charcoal and optionally enzymatic resolution, for example in the presence of the enzyme proteinase 2A, to obtain the (S) enantiomer. According to this method of synthesis, R1, R2, R3, R4 and R5 are as defined above.

The compounds of formula (I) can also be obtained according to the reaction scheme presented by the synthetic route **2b** of **Figure 2** by the reaction of 4(S)-benzyloxazolidin-2-one and of a 2-alkoxyacetic acid chloride for the preparation of an Evans derivative, followed by the condensation of such an Evans derivative, of well-defined chirality, with the aldehyde derivative **15** in the presence of dibutylboron triflate, for example, which results in the derivative **19.** The deoxygenation of compound **19** by the Barton reaction results in compound **20.** Compound **(I)** is obtained from compound **20** by saponification, for example in the presence of lithium hydroxide, or by transesterification, for example with sodium methoxide in methanol. According to this method of synthesis, R1, R2, R3, R4 and R5 are as defined above.

According to the present invention, the term "Evans derivative" is understood to mean, preferably, an oxazolidin-2-one derivative of well-defined chirality, such as a 4(S)-benzyloxazolidin-2-one derivative.

According to the present invention, the term "Barton reaction" is understood to mean the reaction of phenyl chlorothionoformate with the hydroxyl group of compound **19**, in the case of the synthetic route 2b considered in Figure 2, followed by a radical reaction in the presence of tributyltin hydride.

The compounds according to the invention exhibit modulatory properties with regard to receptors of PPAR type. This activity on PPARα, δ and γ receptors is measured in a transactivation test and quantified by the apparent dissociation constant (KdApp), as described in
Example 7 below.

In a manner not obvious to a person skilled in the art in the light of the prior art, the preferred compounds according to the invention exhibit a surprising biological activity, in particular a binding affinity for PPARδ receptors which is significantly increased with respect to that of the compounds according to Patent FR 2 812 876. The KdApp values of the compounds according to the present invention for PPARγ receptors are listed in Example 7 and are illustrated in **Table 1**, where they are compared with those of the compounds of Patent Application FR 2 812 876: it is apparent that they are less than 1 nM and advantageously less than 0.1 nM, i.e. at least 120 times lower and up to several thousand times lower than the KdApp values described for the compounds of Patent FR 2 812 876 (**Table 1**), reflecting a considerably increased affinity of the compounds according to the present invention for PPARγ receptors. In **Table 1**, the KdApp values, with PPARγ receptors, of some compounds according to the invention are lined up with some compounds according to the prior art, the compounds exhibiting similar substituents being considered. In particular, the R1 radical according to the invention is equivalent to the R10 radical of the compounds according to FR 2 812 876 and the R2 radical according to the invention is equivalent to the R4 radical of the compounds according to FR 2 812 876.

In particular, the compounds according to the invention are modulators of specific receptors of PPARγ type, that is to say that they exhibit a ratio of the KdApp for the PPARα or PPARδ receptors to the KdApp for the PPARγ receptors of greater than or equal to 10. Preferably, this PPARα/PPARγ or PPARδ/PPARγ ratio is greater than or equal to 50 and more advantageously greater than or equal to 100.

Another subject-matter of the present invention is the compounds of formula (I) as described above as medicament.

The compounds according to the invention are particularly well suited to the following treatments:
1) of dermatological conditions linked to a disorder of keratinization involving differentiation and proliferation, in particular for treating acne vulgaris, comedonic or polymorphic acne, acne rosacea, nodulocystic acne, acne conglobata, senile acne and secondary acnes, such as solar, drug or occupational acne,
2) of other types of disorders of keratinization, in particular ichthyoses, ichthyosiform conditions, Darier's disease, palmoplantar keratoderma, leucoplakia and leucoplakiform conditions or cutaneous or mucosal (oral) lichen,
3) of other dermatological conditions with an inflammatory immunoallergic component, with or without cell proliferation disorder, and, in particular, all forms of psoriasis, whether cutaneous, mucosal or ungual, and even psoriatic rheumatism, or alternatively cutaneous atopy, such as eczema, or respiratory atopy or alternatively gingival hypertrophy,
4) of all dermal or epidermal proliferations, whether they are benign or malignant and whether they are or are not of viral origin, such as common warts, flat warts and epidermodysplasia verruciformis, florid or oral papillomatoses, T lymphoma, and the proliferations which can be induced by ultraviolet radiation, in particular in the case of basal cell and prickle cell epithelioma, and also all precancerous skin lesions, such as keratoacanthomas,
5) of other dermatological disorders, such as immune dermatoses, such as lupus erythematosus, immune bullous diseases and collagen diseases, such as scleroderma,
6) of dermatological or general conditions with an immunological component,
7) of skin disorders due to exposure to UV radiation, and also for repairing or combating skin ageing, whether photoinduced or chronologic or for reducing actinic keratoses and pigmentations or any pathology associated with chronologic or actinic ageing, such as xerosis,
8) of disorders of the sebaceous function, such as hyperseborrhoea of acne or simple seborrhoea,
9) or the prevention of disorders of cicatrization or the prevention or the repair of stretch marks,
10) of disorders of pigmentation, such as hyperpigmentation, melasma, hypopigmentation or vitiligo,
11) of conditions of the metabolism of lipids, such as obesity, hyperlipidaemia or non-insulin-dependent diabetes,
12) of inflammatory conditions, such as arthritis,
13) or the prevention of cancerous or precancerous conditions,
14) or the prevention of alopecia of various origins, in particular alopecia due to chemotherapy or to radiation,
15) of disorders of the immune system, such as asthma, type I diabetes mellitus, multiple sclerosis or other selective dysfunctions of the immune system,
16) of conditions of the cardiovascular system, such as arteriosclerosis or hypertension.

Another subject-matter of the present invention is a pharmaceutical or cosmetic composition comprising, in a physiologically acceptable medium, at least one compound of formula (I) as defined above.

Another subject-matter of the present invention is the use of the compounds of formula (I) in the manufacture of a composition intended for the treatment of the abovementioned conditions, in particular for regulating and/or restoring the metabolism of skin lipids.

The composition according to the invention can be administered orally, parenterally, topically or ocularly. Preferably, the pharmaceutical composition is packaged in a form suitable for topical application.

Orally, the composition, more particularly the pharmaceutical composition, can be provided in the form of tablets, including sugar-coated tablets, hard gelatin capsules, syrups, suspensions, solutions, powders, granules, emulsions or lipid or polymeric microspheres or nanospheres or vesicles which make possible controlled release. Parenterally, the composition can be provided in the form of solutions or suspensions for infusion or for injection.

The compounds according to the invention are generally administered at a daily dose of approximately 0.001 mg/kg to 100 mg/kg of body weight, taken 1 to 3 times.

The compounds are used systemically at a concentration generally of between 0.001% and 10% by weight, preferably between 0.01% and 1% by weight, with respect to the weight of the composition.

Topically, the pharmaceutical composition according to the invention is more particularly intended for the treatment of the skin and mucous membranes and can be provided in the form of salves, creams, milks, ointments, powders, impregnated pads, solutions, gels, sprays, lotions or suspensions. It can also be provided in the form of lipid or polymeric microspheres or nanospheres or vesicles or of polymeric patches and of hydrogels which make possible controlled release. This topical composition can be provided in the anhydrous form, in the aqueous form or in the form of an emulsion.

The compounds are used topically at a concentration generally of between 0.001% and 10% by weight, preferably between 0.01% and 1% by weight, with respect to the total weight of the composition.

The compounds of formula (I) according to the invention also have an application in the cosmetics field, in particular in body and hair hygiene and more particularly for regulating and/or restoring the metabolism of skin lipids. In comparison with the products known previously, these compounds of formula (I) have the advantage of additionally exhibiting other advantageous properties, in particular antiinflammatory or soothing properties, which makes them less irritating and therefore better tolerated compounds.

Another subject-matter of the invention is thus the cosmetic use of a composition comprising, in a physiologically acceptable vehicle, at least one of the compounds of formula (I) for body or hair hygiene.

The cosmetic composition according to the invention, comprising, in a cosmetically acceptable vehicle, at least one compound of formula (I) or one of its optical or geometrical isomers or one of its salts, can be provided in particular in the form of a cream, a milk, a lotion, a gel, lipid or polymeric microspheres or nanospheres or vesicles, a soap or a shampoo.

The concentration of compound of formula (I) in the cosmetic composition is between 0.001% and 3% by weight, with respect to the total weight of the composition.

The compositions as described above can, of course, additionally comprise inert or even pharmacodynamically active additives or combinations of these additives and in particular: wetting agents; depigmenting agents, such as hydroquinone, azelaic acid, caffeic acid or kojic acid; emollients; moisturizing agents, such as glycerol, polyethylene glycol (PEG) 400, thiamorpholinone and its derivatives, or urea; antiseborrhoeic or antiacne agents, such as S-carboxymethylcysteine, S-benzylcysteamine, their salts or their derivatives, or benzoyl peroxide; antifungal agents, such as ketoconazole or 4,5-polymethylene-3-isothiazolidones; antibacterials; carotenoids and in particular β-carotene; antipsoriatic agents, such as anthralin and its derivatives; eicosa-5,8,11,14-tetraynoic and eicosa-5,8,11-triynoic acids, their esters and amides; and, finally, retinoids. The compounds of formula (I) can also be combined with vitamins D or their derivatives, with corticosteroids, with agents for combating free radicals, with α-hydroxy or α-keto acids or their derivatives, or with ion-channel blockers.

These compositions can also comprise flavour enhancers, preservatives, such as esters of para-hydroxybenzoic acid, stabilizing agents, moisture-regulating agents, pH-regulating agents, agents for modifying osmotic pressure, emulsifying agents, UV-A and UV-B screening agents, or antioxidants, such as α-tocopherol, butylated hydroxyanisole or butylated hydroxytoluene.

Of course, a person skilled in the art will take care to choose the optional compound or compounds to be added to these compositions so that the advantageous properties intrinsically attached to the present invention are not, or not substantially, detrimentally affected by the envisaged addition.

Another subject-matter of the invention relates to a cosmetic process for rendering the skin more attractive, **characterized in that** a composition comprising at least one compound of formula (I) as defined above is applied to the skin. The regulation and/or the restoration of the metabolism of skin lipids makes it possible to obtain skin with a surface appearance which has been rendered more attractive.

Several examples of the preparation of active compounds of formula (I) according to the invention, along with results of biological activities of such compounds and various practical formulations based on these compounds, will now be given, by way of illustration and without any limiting nature.

### Example 1: 2(S)-Ethoxy-3-[3'-(3-heptyl-1-methylureido)biphenyl-4-yl]propanoic acid

### a) tert-Butyl (3-bromophenyl)carbamate

120 g (549 mmol) of di(tert-butyl) dicarbonate are added in small amounts at ambient temperature to a mixture of 94 g (549 mmol) of 3-bromoaniline and 1 1 of dichloromethane. After stirring for 18 hours, the reaction mixture is poured into ice-cold water and extracted with dichloromethane. The organic phase is separated by settling, dried over magnesium sulphate and evaporated. 138 g of tert-butyl (3-bromobenzyl)carbamate are obtained. Yield = 98%.

### b) tert-Butyl (3-bromophenyl)-N-methylcarbamate

19 g (475 mmol) of sodium hydride (60% in oil) are added in small amounts to a solution of 114 g (447 mmol) of tert-butyl (3-bromobenzyl)carbamate in 800 ml of dimethylformamide and the reaction medium is stirred until evolution of gas has ceased. 29.3 ml (470 mmol) of methyl iodide are added dropwise and stirring is maintained for 18 hours. The reaction medium is poured into ice-cold water and extracted with ethyl acetate. The organic phase is separated by settling, dried over magnesium sulphate and evaporated. 115 g of tert-butyl (3-bromobenzyl)-N-methylcarbamate are obtained. Yield = 95%.

### c) tert-Butyl (4'-formylbiphenyl-3-yl)methylcarbamate

307 ml (615 mmol) of an aqueous potassium carbonate solution (2M) are added dropwise to a mixture of 61.5 g (205 mmol) of tert-butyl (3-bromobenzyl)-N-methylcarbamate, 46 g (307 mmol) of 4-formylbenzene-boronic acid and 500 ml of toluene. The reaction medium is subsequently degassed with argon and 7 g (6.2 mmol) of tetrakis(triphenylphosphine)palladium(0) are added. After heating at 90°C for 24 hours, the reaction medium is poured into water and extracted with ethyl acetate. The organic phase is separated by settling, dried over magnesium sulphate and evaporated. The residue obtained is purified by chromatography on a silica column eluted with a mixture of heptane and ethyl acetate (70/30). After evaporating the solvents, 67 g of tert-butyl (4'-formylbiphenyl-3-yl)methylcarbamate are collected. Yield = 60%.

### d) tert-Butyl {4'-[3-(4(S)-benzyl-2-oxo-oxazolidin-3-y1)-2(S)-ethoxy-1(R)-hydroxy-3-oxopropyl]-biphenyl-3-yl}methylcarbamate

72.3 ml (72.3 mmol) of dibutylboron triflate and then 12.6 ml (72.3 mmol) of diisopropylethylamine are added dropwise to a solution, cooled to 0°C, of 15.2 g (57.8 mmol) of (S)-4-benzyl-3-(2-ethoxyacetyl)-oxazolidin-2-one, prepared as described in the publication by Bernard Hulin et al., J. Med. Chem., 1996, 39, 3897-3907, from commercial (S)-4-benzyl-oxazolidin-2-one, in 150 ml of dichloromethane. The reaction medium is stirred at 0°C for 30 min and then cooled to -78°C. A solution of 15 g (48.2 mmol) of tert-butyl (4'-formylbiphenyl-3-yl)methylcarbamate in 70 ml of dichloromethane is then added dropwise. After stirring from -78°C to ambient temperature over 4 hours, the reaction medium is cooled to 0°C and a mixture of 130 ml of a buffer solution, pH = 7, and of 100 ml of methanol is added dropwise, followed by the dropwise addition of a mixture of 130 ml of aqueous hydrogen peroxide solution and of 100 ml of methanol. The reaction medium is stirred at 0°C for 1 hour and then at ambient temperature for 3 hours. After addition of water, the reaction medium is extracted with dichloromethane. The organic phase is dried over magnesium sulphate, filtered and evaporated under vacuum. The residue obtained is purified by chromatography on a silica column eluted with a mixture of heptane and ethyl acetate (70/30) and then increase in the polarity up to a 50/50 heptane/ethyl acetate mixture. After evaporation of the solvents, 28 g of tert-butyl {4'-[3-(4(*S*)-benzyl-2-oxooxazolidin-3-yl)-2(*S*)-ethoxy-1(R)-hydroxy-3-oxopropyl]biphenyl-3-yl}methylcarbamate are collected. Yield = 81%.

### e) tert-Butyl (S)-{4'-[3-(4-benzyl-2-oxo-oxazolidin-3-yl)-2-ethoxy-3-oxopropyl]biphenyl-3-yl}-methylcarbamate

4.8 ml (9.6 mmol) of sodium bis(trimethylsilylamide) are added dropwise to a solution, cooled beforehand to 0°C, of 5 g (8.7 mmol) of tert-butyl {4'-[3-(4(*S*)-benzyl-2-oxooxazolidin-3-yl)-2(*S*)-ethoxy-1(R)-hydroxy-3-oxopropyl]biphenyl-3-yl}methylcarbamate in 70 ml of tetrahydrofuran. The reaction medium is stirred at -78°C for 1 hour, then 1.3 ml (9.6 mmol) of phenyl chlorothionoformate are added and the medium is stirred at -78°C for 1 hour and then at ambient temperature for 1 hour 30 min. After evaporation of the tetrahydrofuran, the reaction medium is extracted with dichloromethane and washed with water. The organic phase is separated by settling, dried over magnesium sulphate, filtered and evaporated under vacuum. The 9 g (8.7 mmol) of residue obtained are placed in 100 ml of toluene and 71 mg (0.4 mmol) of 2,2'-azobis(2-methylpropionitrile) and then 3.5 ml (13.1 mmol) of tributyltin hydride are added. The reaction medium is heated at 110°C for 20 minutes. After addition of water, the reaction medium is extracted with ethyl acetate. The organic phase is washed with water and with a saturated aqueous sodium chloride solution, dried over magnesium sulphate, filtered and evaporated. The residue obtained is purified by chromatography on a silica column eluted with a mixture of heptane and ethyl acetate (90/10) and then increase in the polarity up to a 70/30 heptane/ethyl acetate mixture. After evaporation of the solvents, 2.85 g of tert-butyl (S)-{4'-[3-(4-benzyl-2-oxooxazolidin-3-yl)-2-ethoxy-3-oxopropyl]biphenyl-3-yl}methylcarbamate are obtained. Yield = 60%.

### f) 4(S)-Benzyl-3-[2(S)-ethoxy-3-(3'-(methylamino)biphenyl-4-yl)propionyl]oxazolidin-2-one

9 ml (114 mmol) of trifluoroacetic acid are added dropwise to a solution of 8.5 g (15.2 mmol) of (S)-{4'-[3-(4-benzyl-2-oxooxazolidin-3-yl)-2-ethoxy-3-oxopropyl]biphenyl-3-yl}methylcarbamate in 150 ml of dichloromethane. The reaction medium is stirred at ambient temperature for 24 h, added to water and extracted with dichloromethane. The organic phase is dried over magnesium sulphate, filtered and evaporated. 8.7 g of 4 (*S*)-benzyl-3-[2(*S*)-ethoxy-3-(3'-(methylamino)biphenyl-4-yl)propionyl]oxazolidin-2-one are obtained in the form of a trifluoroacetate salt. Yield = 100%.

### g) 1-{4'-[3-(4(S)-Benzyl-2-oxooxazolidin-3-yl)-2(S)-ethoxy-3-oxopropyl]biphenyl-3-yl}-3-heptyl-1-methylurea

1.1 ml (7.7 mmol) of triethylamine and then 2.25 ml (14.0 mmol) of heptyl isocyanate are added dropwise to a solution of 4 g (7.0 mmol) of 4(*S*)-benzyl-3-[2(*S*)-ethoxy-3-(3'-(methylamino)biphenyl-4-yl)propionyl]oxazolidin-2-one in 50 ml of dichloromethane. After stirring at ambient temperature for 20 hours, the reaction medium is placed in water and extracted with dichloromethane. The organic phase is dried over magnesium sulphate, filtered and evaporated. The residue obtained is purified by chromatography on a silica column eluted with a mixture of heptane and ethyl acetate (50/50). After evaporation of the solvents, 3.6 g of 1-{4'-[3-(4(*S*)-benzyl-2-oxo-oxazolidin-3-yl)-2(*S*)-ethoxy-3-oxopropyl]biphenyl-3-yl}-3-heptyl-1-methylurea are collected in the form of a colourless oil. Yield = 86%.

### h) 2(S)-Ethoxy-3-[3'-(3-heptyl-1-methylureido)biphenyl-4-yl]propanoic acid

18 ml (9.0 mmol) of a 0.5M aqueous lithium hydroxide solution are added to a solution, cooled beforehand to 0°C, of 3.6 g (6.0 mmol) of 1-{4'-[3-(4(*S*)-benzyl-2-oxooxazolidin-3-yl)-2(*S*)-ethoxy-3-oxopropyl]biphenyl-3-yl}-3-heptyl-1-methylurea in 80 ml of tetrahydrofuran. The reaction medium is stirred at 0°C for 2 hours, then a portion of the tetrahydrofuran is evaporated, and water and n-butanol are added. The reaction medium is acidified with a 1N hydrochloric acid solution to pH 3 and extracted with n-butanol. The organic phase is dried over magnesium sulphate, filtered and evaporated under vacuum. The residue obtained is purified by chromatography on a silica column eluted with a mixture of heptane and ethyl acetate (70/30) and then increase in the polarity up to a 50/50 heptane/ethyl acetate mixture. After evaporation of the solvents, 1.5 g of 2(*S*)-ethoxy-3-[3'-(3-heptyl-1-methylureido)biphenyl-4-yl]propanoic acid are collected in the form of a colourless oil. Yield = 57%.

¹H NMR (δ, CDCl₃): 0.87 (t, J = 7 Hz, 3H); 1.20-1.24 (m, 8H), 1.43 (m, 2H), 3.12 (m, 1H), 3.18 (m, 1H), 3.22 (m, 2H), 3.32 (s, 3H), 3.49 (m, 1H), 3.69 (m, 1H), 4.15 (m, 1H), 4.43 (m, 1H), 7.22-7.56 (m, 8H).

### i) L-Arginine salt of 2(S)-ethoxy-3-[3'-(3-heptyl-1-methylureido)biphenyl-4-yl]propanoic acid

An aqueous solution of 0.4 g (2.3 mmol) of L-arginine is added dropwise to a solution, heated beforehand to 78°C, of 1 g (2.3 mmol) of 2(*S*)-ethoxy-3-[3'-(3-heptyl-1-methylureido)biphenyl-4-yl]propanoic acid in 22 ml of ethanol. The reaction medium is heated at 78°C for 1 hour, then it is brought back to ambient temperature overnight and evaporated to dryness under vacuum. The residue obtained is taken up in 15 ml of ethyl ether, stirred at ambient temperature for 30 min and filtered off. The solid obtained is rinsed with ethyl ether and dried under vacuum in an oven. 1.3 g of the L-arginine salt of 2(*S*)-ethoxy-3-[3'-(3-heptyl-1-methylureido)biphenyl-4-yl]propanoic acid are obtained in the form of a white powder. Yield = 100%.

¹H NMR (δ, d₆-DMSO)_{:} 0.84 (m, 3H), 1.00 (m, 3H), 1.22 (m, 8H), 1.37 (m, 2H), 1.27-1.39 (m, 4H), 2.90 (m, 1H), 2.97-3.07 (m, 3H), 3.18 (s, 3H), 3.20 (m, 1H), 3.60 (m, 1H), 3.67 (m, 1H), 6.05 (m, 1H), 7.18-7.54 (m, 8H).

### Example 2: 2(S)-Ethoxy-3-[3'-(1-methyl-3-pentylureido)biphenyl-4-yl]propanoic acid

### a) 1-{4'-[3-(4(S)-Benzyl-2-oxooxazolidin-3-yl)-2(S)-ethoxy-3-oxopropyl]biphenyl-3-yl}-1-methyl-3-pentylurea

In a manner analogous to Example 1g), from 0.8 g (1.4 mmol) of 4(*S*)-benzyl-3-[2(*S*)-ethoxy-3-(3'-(methylamino)biphenyl-4-yl)propionyl]oxazolidin-2-one and 0.35 ml (2.8 mmol) of pentyl isocyanate, 0.54 g of 1-{4'-[3-(4(*S*)-benzyl-2-oxooxazolidin-3-yl)-2(*S*)-ethoxy-3-oxopropyl]biphenyl-3-yl}-1-methyl-3-pentylurea is obtained in the form of a colourless oil. Yield = 67%.

### b) 2(S)-Ethoxy-3-[3'-(1-methyl-3-pentyl-ureido)biphenyl-4-yl]propanoic acid

In a manner analogous to Example 1h), from 0.53 g (0.93 mmol) of 1-{4'-[3-(4(*S*)-benzyl-2-oxo-oxazolidin-3-yl)-2(*S*)-ethoxy-3-oxopropyl]biphenyl-3-yl}-1-methyl-3-pentylurea and 2.8 ml (1.4 mmol) of a 0.5N aqueous sodium hydroxide solution, 0.32 g of 2(*S*)-ethoxy-3-[3'-(1-methyl-3-pentylureido)biphenyl-4-yl]-propanoic acid is obtained in the form of a colourless oil. Yield = 84%.

¹H NMR (δ, CDCl₃): 0.87 (t, J = 7 Hz, 3H), 1.21 (t, J = 7 Hz, 3H), 1.25-1.37 (m, 8H), 1.60 (m, 2H), 3.11 (dd, J = 7.8 Hz, J = 14 Hz, 1H), 3.38 (dd, J = 7 Hz, J = 14 Hz, 1H), 3.40 (m, 2H), 3.47 (m, 3H), 3.50 (m, 1H), 3.65 (m, 1H), 4.15 (m, 1H), 6.94 (d, J = 8.4 Hz, 1H), 7.35-7.40 (m, 3H), 7.75-7.82 (m, 3H).

### c) L-Arginine salt of 2(S)-ethoxy-3-[3'-(1-methyl-3-pentylureido)biphenyl-4-yl]propanoic acid

In a manner analogous to Example 1i), from 0.32 g (0.8 mmol) of 2(*S*)-ethoxy-3-[3'-(1-methyl-3-pentylureido)biphenyl-4-yl]propanoic acid and 0.13 g (0.8 mmol) of arginine, 0.45 g of the L-arginine salt of 2(*S*)-ethoxy-3-[3'-(1-methyl-3-pentylureido)biphenyl-4-yl]propanoic acid is obtained in the form of a white solid. Yield = 100%.

¹H NMR (δ, d₆-DMSO): 0.86 (t, J = 7 Hz, 3H), 1.01 (t, J = 7 Hz, 3H), 1.20-1.28 (m, 8H), 1.30 (m, 2H), 1.40 (m, 2H), 1.41-1.57 (m, 2H), 2.8 (m, 1H), 3.00-3.10 (m, 4H), 3.20 (s, 3H), 3.22 (m, 1H), 3.33 (m, 1H), 3.42 (m, 1H), 3.67 (m, 1H), 6.06 (m, 1H), 7.19-7.55 (m, 8H).

### Example 3: 2(S)-cyclopropylmethoxy-3-[3'-(3-heptyl-1-methylureido)biphenyl-4-yl]propanoic acid

a) 3.6 g (12.7 mmol) of tert-butyl (3-bromophenyl)-N-methylcarbamate, prepared in a manner analogous to Example 1b), are dissolved in 15 ml of dichloromethane. 5 ml of trifluoroacetic acid are added and the reaction mixture is stirred at ambient temperature for 1 hour. The reaction is halted by the addition of 50 ml of a saturated sodium hydrogencarbonate solution and then extraction is carried out with ethyl acetate. The organic phases are combined and dried over sodium sulphate. The solvents are evaporated and then the residue is chromatographed on silica gel (heptane/ethyl acetate 50/50). 2.14 g of 3-bromo-N-methylaniline are obtained in the form of an oil. Yield = 90%.
b) 890 mg (3.5 mmol) of pinacolborane are added to a mixture of 600 mg (3.2 mmol) of 3-bromo-N-methylaniline and 1 g (10.2 mmol) of potassium acetate in the presence of 130 mg (0.16 mmol, 5 mol%) of palladium dichloride diphenylphosphinopropane ferrocene (PdCl₂dppf) in 10 ml of dimethylformamide. The mixture is stirred at 90°C for 2 hours. The reaction is halted by the addition of 20 ml of water and then extraction is carried out with ethyl acetate. The organic phases are combined and dried over sodium sulphate. The solvents are evaporated and then the residue is chromatographed on silica gel (heptane/ethyl acetate 80/20). 420 mg of methyl[3-(4,4,5,5-tetramethyl-1,3,2-dioxaborolan-2-yl)phenyl]amine are obtained in the form of an oil. Yield = 57%.
c) 72 ml (0.122 mol, 2.5 eq) of *tert*-butyllithium (1.7M/pentane) are added slowly using a needle to a suspension of 35 g (0.148 mol, 3 eq) of 1,4-dibromobenzene in 100 ml of tert-butyl methyl ether at -30°C. The mixture is stirred at -30°C for 10 min and then 5.3 g (0.059 mol) of copper(I) cyanide are introduced into the above solution. The reaction mixture is stirred at -30°C for 20 min. A solution of 5 g (0.049 mol) of methyl (*S*)-glycidate in 10 ml of tert-butyl methyl ether is added while keeping the temperature below -20°C. The mixture is stirred at -30°C for 20 min and then the reaction is halted by the addition of a saturated ammonium chloride solution. The mixture is extracted with 3 × 300 ml of ethyl acetate. The organic phases are combined and dried over sodium sulphate. The solvents are evaporated and then the residue is chromatographed on silica gel (heptane 100% up to heptane/ethyl acetate 60/40). 7.2 g of methyl (*S*)-3-(4-bromophenyl)-2-hydroxypropionate are obtained in the form of a solid. Yield = 56%.
d) 0.11 ml (1.15 mmol) of bromomethylcyclopropane are added to a mixture of 267 mg (3.48 mmol) of silver oxide and 100 mg (0.38 mmol) of methyl (*S*)-3-(4-bromophenyl)-2-hydroxypropionate in 2 ml of diethyl ether. The reaction mixture is stirred at 50°C for 24 hours. The mixture is filtered and then the solvents are evaporated. The residue is chromatographed on silica gel (heptane/ethyl acetate 85/15). 145 mg of methyl (*S*)-3-(4-bromophenyl)-2-(cyclopropylmethoxy)-propanoate are obtained in the form of an oil. Yield = 50%.
e) 53 mg (0.046 mmol) of tetrakis(triphenylphosphine)palladium are added to a solution of 145 mg (0.46 mmol) of methyl (*S*)-3-(4-bromophenyl)-2-(cyclopropylmethoxy)propionate and 129 mg (0.55 mmol) of methyl[3-(4,4,5,5-tetramethyl-1,3,2-dioxaborolan-2-yl)phenyl]amine in 3 ml of dimethylformamide. 0.3 ml of a 2M potassium phosphate solution is added and the reaction mixture is stirred at 90°C for 2 hours. The reaction is halted by the addition of 10 ml of water and then extraction is carried out with ethyl acetate. The organic phases are combined and dried over sodium sulphate. The solvents are evaporated and then the residue is chromatographed on silica gel (heptane/ethyl acetate 80/20). 70 mg of methyl (*S*)-2-cyclopropylmethoxy-3-[3'-(methylamino)biphenyl-4-yl]propanoate are obtained in the form of an oil. Yield = 45%.
f) 40 µl (0.25 mmol) of heptyl isocyanate are added to a solution of 70 g (0.2 mmol) of methyl (*S*)-2-cyclopropylmethoxy-3-[3'-(methylamino)biphenyl-4-yl]-propionate in 2 ml of dichloromethane. The reaction mixture is stirred at ambient temperature for 48 hours. The reaction is halted by the addition of 2 ml of water and then extraction is carried out with ethyl acetate. The organic phases are combined and dried over sodium sulphate. The solvents are evaporated and then the residue is chromatographed on silica gel (heptane/ethyl acetate 70/30). 86 mg of methyl (*S*)-2-cyclopropylmethoxy-3-[3'-(3-heptyl-1-methylureido)biphenyl-4-yl]-propanoate are obtained in the form of an oil. Yield = 87%.
g) 21 mg (0.54 mmol) of sodium hydroxide are added to a solution of 86 mg (0.18 mmol) of methyl (*S*)-2-cyclopropylmethoxy-3-[3'-(3-heptyl-1-methylureido)biphenyl-4-yl]propionate in 2 ml of 9/1 tetrahydrofuran/methanol. The reaction mixture is stirred at ambient temperature overnight. The reaction is halted by the addition of 2 ml of water and 0.5 ml of acetic acid, and then extraction is carried out with ethyl acetate. The organic phases are combined and dried over sodium sulphate. The solvents are evaporated and then the residue is chromatographed on silica gel (dichloromethane/methanol 90/10). 70 mg of 2(*S*)-cyclopropylmethoxy-3-[3'-(3-heptyl-1-methylureido)biphenyl-4-yl]propanoic acid are obtained in the form of an oil. Yield = 84%.

¹H NMR: (CDCl₃, 400 MHz): 0.20 (m, 2H), 0.56 (m, 2H), 0.86 (t, *J* = 6.8 Hz, 3H), 1.05 (m, 1H), 1.24 (m, 8H), 1.42 (m, 2H), 3.07-3.25 (m, 4H), 3.32 (s, 3H), 3.39 (m, 2H), 4.20 (dd, *J* = 4, 7.6 Hz, 1H), 4.40 (t, *J* = 5.6 Hz, 1H), 7.22 (d, *J* = 7.6 Hz, 1H), 7.38 (d, *J* = 8.4 Hz, 2H), 7.47-7.54 (m, 5H).

### Example 4: 2-(S)-Propyloxy-3-[3'-(3-heptyl-1-methylureido)biphenyl-4-yl]propanoic acid

a) 0.22 ml (2.31 mmol) of propyl iodide is added to a mixture of 793 mg (3.48 mmol) of silver oxide and 300 mg (1.16 mmol) of methyl (*S*)-3-(4-bromophenyl)-2-hydroxypropionate in 3 ml of diethyl ether. The reaction mixture is stirred at 50°C for 12 hours. The mixture is filtered and then the solvents are evaporated. The residue is chromatographed on silica gel (heptane/ethyl acetate 80/20). 291 mg of methyl (*S*)-3-(4-bromophenyl)-2-(propyloxy)propanoate are obtained in the form of an oil. Yield = 83%.
b) 38 mg (0.033 mmol) of tetrakis(triphenylphosphine)palladium are added to a solution of 100 mg (0.33 mmol) of methyl (*S*)-3-(4-bromophenyl)-2-(propyloxy)propionate and 90 mg (0.39 mmol) of methyl[3-(4,4,5,5-tetramethyl-1,3,2-dioxaborolan-2-yl)phenyl]-amine in 1 ml of dimethylformamide. 0.2 ml of a 2M potassium phosphate solution is added and the reaction mixture is stirred at 90°C for 2 hours. The reaction is halted by the addition of 10 ml of water and then extraction is carried out with ethyl acetate. The organic phases are combined and dried over sodium sulphate. The solvents are evaporated and then the residue is chromatographed on silica gel (heptane/ethyl acetate 80/20). 76 mg of methyl (*S*)-2-propyloxy-3-[3'-(methylamino)biphenyl-4-yl]propanoate are obtained in the form of an oil. Yield = 70%.
c) 156 µl (0.96 mmol) of heptyl isocyanate are added to a solution of 210 mg (0.64 mmol) of methyl (*S*)-2-propyloxy-3-[3'-(methylamino)biphenyl-4-yl]-propanoate in 3 ml of dichloromethane. The reaction mixture is stirred at ambient temperature for 48 hours. The reaction is halted by the addition of 2 ml of water and then extraction is carried out with ethyl acetate. The organic phases are combined and dried over sodium sulphate. The solvents are evaporated and then the residue is chromatographed on silica gel (heptane/ethyl acetate 70/30). 200 mg of methyl (*S*)-2-propyloxy-3-[3'-(3-heptyl-1-methylureido)biphenyl-4-yl]propanoate are obtained in the form of an oil. Yield = 66%.
d) 51 mg (1.28 mmol) of sodium hydroxide are added to a solution of 200 mg (0.42 mmol) of methyl (*S*)-2-propyloxy-3-[3'-(3-heptyl-1-methylureido)-biphenyl-4-yl]propanoate in 2 ml of 9/1 tetrahydrofuran/methanol. The reaction mixture is stirred overnight at ambient temperature. The reaction is halted by the addition of 2 ml of water and 0.5 ml of acetic acid, and then extraction is carried out with ethyl acetate. The organic phases are combined and dried over sodium sulphate. The solvents are evaporated and then the residue is chromatographed on silica gel (dichloromethane/methanol 90/10). 147 mg of 2-(*S*)-propyloxy-3-[3'-(3-heptyl-1-methylureido)biphenyl-4-yl]propanoic acid are obtained in the form of an oil. Yield = 76%.

¹H NMR (CDCl₃, 400 MHz): 0.86 (t, *J* = 7.1 Hz, 3H), 0.90 (t, *J* = 7.2 Hz, 3H), 1.24 (m, 8H), 1.43 (m, 2H), 1.61 (sext, *J* = 7 Hz, 2H), 3.07-3.22 (m, 4H), 3.32 (s, 3H), 3.38 and 3.57 (2q, *J* = 7.7 Hz, 2H), 4.13 (m, 1H), 4.41 (t, *J* = 5.6 Hz, 1H), 7.22 (d, *J* = 7.6 Hz, 1H), 7.37 (d, *J* = 8.4 Hz, 2H), 7.47-7.55 (m, 5H).

### Example 6: 2(S)-Allyloxy-3-[3'-(3-heptyl-1-methylureido)biphenyl-4-yl]propanoic acid

a) 100 mg of 10% palladium-on-charcoal are added to a solution of 2.4 g (4.66 mmol) of methyl (*S*)-2-benzyloxy-3-[3'-(3-heptyl-1-methylureido)biphenyl-4-yl]propanoate in 10 ml of methanol. The reaction mixture is stirred overnight under a hydrogen atmosphere. The reaction mixture is filtered and then the solvents are evaporated. The residue is filtered through silica gel (ethyl acetate). 1.61 g of methyl (*S*)-3-[3'-(3-heptyl-1-methylureido)biphenyl-4-yl]-2-hydroxypropionate are collected in the form of a colourless oil. Yield = 81%.
b) 58 µl (0.70 mmol) of allyl bromide are added to a mixture of 162 mg (0.70 mmol) of silver oxide and 200 mg (0.47 mmol) of methyl (*S*)-3-[3'-(3-heptyl-1-methylureido)biphenyl-4-yl]-2-hydroxy-propanoate in 3 ml of diethyl ether. The reaction mixture is stirred at 40°C for 24 hours. The mixture is filtered and then the solvents are evaporated. The residue is chromatographed on silica gel (heptane/ethyl acetate 80/20 up to 60/40). 180 mg of methyl (*S*)-2-allyloxy-3-[3'-(3-heptyl-1-methylureido)biphenyl-4-yl]-propanoate are obtained in the form of an oil. Yield = 82%.
c) 56 mg (1.4 mmol, 3 eq) of sodium hydroxide are added to a solution of 200 mg (0.47 mmol, 1 eq) of methyl (*S*)-2-allyloxy-3-[3'-(3-heptyl-1-methylureido)-biphenyl-4-yl]propanoate in 2 ml of 9/1 THF/methanol. The reaction mixture is stirred at ambient temperature for 3 hours. The reaction is halted by the addition of 2 ml of water and 0.5 ml of acetic acid, and then extraction is carried out with ethyl acetate. The organic phases are combined and dried over sodium sulphate. The solvents are evaporated and then the residue is chromatographed on silica gel (dichloromethane/methanol 90/10). 152 mg of 2(*S*)-allyloxy-3-[3'-(3-heptyl-1-methylureido)biphenyl-4-yl]-propanoic acid are obtained in the form of an oil. Yield = 78%.

¹H NMR (CDCl₃, 400 MHz): 0.86 (t, *J* = 6.8 Hz, 3H), 1.26 (m, 8H), 1.42 (m, 2H), 3.09-3.25 (m, 4H), 3.32 (s, 3H), 4.01 (dd, J= 5.8, 12.6 Hz, 1H), 4.16 (dd, *J* = 5.7, 12.6 Hz, 1H), 4.22 (dd, *J* = 4.3, 7.6 Hz, 1H), 4.41 (t, *J* = 5.6 Hz, 1H), 5.22 (d, *J* = 10.4 Hz, 1H), 5.25 (d, *J* = 18.8 Hz, 1H), 5.83 (m, 1H), 7.22 (d, *J* = 7.6 Hz, 1H), 7.37 (d, *J* = 8.4 Hz, 2H), 7.52 (m, 5H).

### Example 7: Crossed-curve PPAR transactivation assay

Activation of the PPAR receptors by an agonist (activator) in HeLN cells leads to the expression of a reporter gene, luciferase, which, in the presence of a substrate, generates light. The modulation of the PPAR receptors is measured by quantifying the luminescence produced after incubation of the cells in the presence of a reference agonist. The ligands will displace the agonist from its site. The measurement of the activity is performed by quantifying the light produced. This measurement makes it possible to determine the modulatory activity of the compounds according to the invention by the determination of the constant which represents the affinity of the molecule for the PPAR receptor. Since this value can fluctuate depending on the basal activity and the expression of the receptor, it is referred to as apparent Kd (KdApp in nM).

To determine this constant, "crossed curves" for the test product, against a reference agonist, are prepared using a 96-well plate: 10 concentrations of the test product plus a concentration 0 are arranged in a line, and 7 concentrations of the agonist plus a concentration 0 are arranged in a column. This represents 88 measurement points for 1 product and 1 receptor. The remaining 8 wells are used for repeatability controls.

In each well, the cells are in contact with a concentration of the test product and a concentration of the reference agonist, 2-(4-{2-[3-(2,4-difluorophenyl)-1-heptylureido]ethyl}phenylsulphanyl)-2-methyl-propionic acid for PPARα, {2-methyl-4-[4-methyl-2-(4-(trifluoromethyl)phenyl)thiazol-5-ylmethylsulphanyl]-phenoxy}acetic acid for PPARδ and 5-{4-[2-(methyl(pyrid-2-yl)amino)ethoxy]benzyl}thiazolidine-2,4-dione for PPARγ. Measurements are also taken for total agonist controls with the same products.

The HeLN cell lines used are stable transfectants containing the plasmids ERE-βGlob-Luc-SV-Neo (reporter gene) and PPAR (α, δ, γ) Gal-hPPAR. These cells are seeded in 96-well plates at the rate of 10 000 cells per well in 100 µl of DMEM medium without phenol red and supplemented with 10% of defatted calf serum. The plates are then incubated for 16 hours at 37°C and 7% CO₂.

The various dilutions of the test products and of the reference ligand are added at the rate of 5 µl per well. The plates are subsequently incubated for 18 hours at 37°C and 7% CO₂, The culture medium is removed by turning over and 100 µl of a 1:1 PBS/luciferin mixture are added to each well. After 5 minutes, the plates are read using the luminescence reader.

These crossed curves make it possible to determine the AC50 values (concentration at which 50% activation is observed) of the reference ligand at various concentrations of test product. These AC50 values are used to calculate the Schild regression by plotting a straight line corresponding to the Schild equation ("Quantitation in Receptor Pharmacology", Terry P. Kenakin, Receptors and Channels, 2001, 7, 371-385) which allows the KdApp values (in nM) to be obtained.

### Transactivation results:

| | PPARα KdApp | PPARδ KdApp | PPARγ KdApp |
|---|---|---|---|
| Compounds | (in nM) | (in nM) | (in nm) |
| Reference 1: 2-(4-{2-[3-(2,4-difluorophenyl)-1-heptylureido]ethyl}phenylsulphany)-2-methyl propionic acid | 200 | n.a. | n.a. |
| Reference 2: {2-methyl-4-[4-methyl-2-(4-(trifluoromethyl)phenyl)thiazol-5-ylmethylsulphanyl]phenoxy}acetic acid | n.a. | 10 | n.a. |
| Reference 3: 5-{4-[2-(methyl(pyridin-2-yl)amino)ethoxy]benzyl}thiazolidine-2,4-dione | n.a. | n.a. | 30 |
| Example 1: 2(S)-ethoxy-3-[3'-(3-heptyl-1-methylureido)biphenyl-4-yl]propionic acid | 30 | 250 | < 1 |
| Example 2: 2(S)-ethoxy-3-[3'-(1-methyl-3-penylureido)biphenyl-4-yl]propionic acid | 250 | 2000 | 0.03 |
| Example 4: 2-(S)-propyloxy-3-[3'-(3-heptyl-1-methylureido)biphenyl-4-yl]propionic acid | 30 | 500 | 0.025 |
| Example 6: 2-(S)-allyloxy-3-[3'-(3-heptyl-1-methylureido)biphenyl-4-yl]propionic acid | 2 | 50 | 0.003 |

| | | | |
|---|---|---|---|
| n.a. means not active | | | |

These results show the affinity of the compounds for PPARγ and more particularly the specificity of the affinity of the compounds of the invention for the PPARγ subtype, compared with the affinity of the compounds for the PPARα subtype or for the PPARδ subtype.

### Example 8: Compositions

Various practical formulations based on the compounds according to the invention have been illustrated in this example.

### A- ORAL ROUTE

| | (a) 0.2 g tablet | |
|---|---|---|
| - Compound of Example 1 | | 0.001 g |
| - Starch | | 0.114 g |
| - Dicalcium phosphate | | 0.020 g |
| - Silica | | 0.020 g |
| - Lactose | | 0.030 g |
| - Talc | | 0.010 g |
| - Magnesium stearate | | 0.005 g |

| | (b) Suspension to be taken orally in 5 ml vials | |
|---|---|---|
| - Compound of Example 5 | | 0.001 g |
| - Glycerol | | 0.500 g |
| - 70% Sorbitol | | 0.500 g |
| - Sodium saccharinate | | 0.010 g |
| - Methyl para-hydroxybenzoate | | 0.040 g |
| - Flavouring | | q.s. |
| - Purified water | | q.s. for 5 ml |

| | (c) 0.8 g tablet | |
|---|---|---|
| - Compound of Example 2 | | 0.500 g |
| - Pregelatinized starch | | 0.100 g |
| - Microcrystalline cellulose | | 0.115 g |
| - Lactose | | 0.075 g |
| - Magnesium stearate | | 0.010 g |

| | (d) Suspension to be taken orally in 10 ml vials | |
|---|---|---|
| - Compound of Example 4 | | 0.200 g |
| - Glycerol | | 1.000 g |
| - 70% Sorbitol | | 1.000 g |
| - Sodium saccharinate | | 0.010 g |
| - Methyl para-hydroxybenzoate | | 0.080 g |
| - Flavouring | | q.s. |
| - Purified water | | q.s. for 10 ml |

### B- TOPICAL ROUTE

| | (a) Salve | |
|---|---|---|
| - Compound of Example 6 | | 0.020 g |
| - Isopropyl myristate | | 81.700 g |
| - Liquid petrolatum | | 9.100 g |
| - Silica ("Aerosil 200", sold by Degussa) | | 9.180 g |

| | (b) Salve | |
|---|---|---|
| - Compound of Example 2 | | 0.300 g |
| - White petrolatum, pharmaceutical grade | | q.s. for 100 g |

| | (c) Nonionic water-in-oil cream | |
|---|---|---|
| - Compound of Example 1 | | 0.100 g |
| - Mixture of emulsive lanolin alcohols, of waxes and of oils ("Anhydrous eucerin", sold by BDF) | | 39.900 g |
| - Methyl para-hydroxybenzoate | | 0.075 g |
| - Propyl para-hydroxybenzoate | | 0.075 g |
| - Sterile demineralized water | | q.s. for 100 g |

| | (d) Lotion | |
|---|---|---|
| - Compound of Example 3 | | 0.100 g |
| - Polyethylene glycol (PEG) 400 | | 69.900 g |
| - 95% Ethanol | | 30.000 g |

| | (e) Hydrophobic salve | |
|---|---|---|
| - Compound of Example 5 | | 0.300 g |
| - Isopropyl myristate | | 36.400 g |
| - Silicone oil ("Rhodorsil 47 V 300", sold by Rhône-Poulenc) | | 36.400 g |
| - Beeswax | | 13.600 g |
| - Silicone oil ("Abil 300,000 cSt", sold by Goldschmidt) | | q.s. for 100 g |

| | | |
|---|---|---|
| | (f) Nonionic oil-in-water cream | |
| - Compound of Example 2 | | 1.000 g |
| - Cetyl alcohol | | 4.000 g |
| - Glyceryl monostearate | | 2.500 g |
| - PEG 50 stearate | | 2.500 g |
| - Shea butter | | 9.200 g |
| - Propylene glycol | | 2.000 g |
| - Methyl para-hydroxybenzoate | | 0.075 g |
| - Propyl para-hydroxybenzoate | | 0.075 g |
| - Sterile demineralized water | | q.s. for 100 g |

## Claims

1. Compound of formula (I): 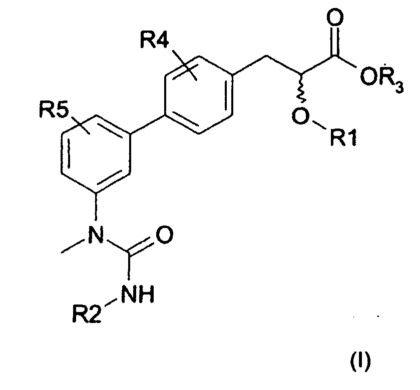 in which:
- R1 represents an alkyl group having 1 to 6 carbon atoms chosen from the methyl, ethyl, n-propyl, isopropyl, isobutyl, tert-butyl, n-pentyl, isopentyl, n-hexyl, and isohexyl radicals; a cyclopropyl, a cyclohexyl, an ethylenyl, an allyl, a propenyl, a butenyl, a pentenyl or an hexenyl radical, an acetyl group, a methylcyclopropane group, an aralkyl group or an aryl group, the aryl group being chosen from a biphenyl, cinnamyl or naphthyl radical which can be mono- or disubstituted by a halogen atom, a CF₃ radical, an alkyl radical having from 1 to 6 carbon atoms, an alkoxy radical having from 1 to 6 carbon atoms, a nitro functional group, a polyether radical, an aryl radical, a benzoyl radical, an alkyl ester group, a carboxylic acid, a hydroxyl radical optionally protected by an acetyl or benzoyl group, or an amino functional group optionally protected by an acetyl or benzoyl group or optionally substituted by at least one alkyl having from 1 to 6 carbon atoms, and the aralkyl group being chosen from a phenethyl or naphth-2-ylmethyl radical which is unsubstituted or substituted by one or more radicals chosen from a halogen atom, a CF₃ radical, an alkyl radical having from 1 to 6 carbon atoms, an alkoxy radical having from 1 to 6 carbon atoms, a hydroxyl radical or an amino functional group which is unprotected or unsubstituted or optionally substituted by at least one alkyl having from 1 to 6 carbon atoms, or a carboxyl functional group;
- R2 represents an alkyl group having 3 to 8 carbon atoms, or a cyclopropyl, a cyclopentyl, a cyclohexyl, an allyl, a propenyl, a butenyl, a pentenyl, an hexenyl, an heptenyl or an octenyl radical;
- R3 is a hydrogen atom;
- R4 and R5 represent a hydrogen atom;
and the possible isomers, optical and/or geometrical, pure or as a mixture, in all proportions, of the said compound of formula (I) and the possible tautomeric forms, and also the salts of the said compound of formula (I).

2. Compound according to Claim 1, **characterized in that** it is provided in the form of an alkali metal salt or of an alkaline earth metal salt or of an organic amine salt.

3. Compound according to Claim 2, **characterized in that** it is provided in the form of a sodium salt.

4. Compound according to Claim 2, **characterized in that** it is provided in the form of an amino acid salt.

5. Compound according to Claim 4, **characterized in that** it is provided in the form of an arginine salt or of a lysine salt.

6. Compound according to one of Claims 1 to 5, **characterized in that** the alkyl radicals having from 1 to 6 carbon atoms are chosen from the methyl, ethyl, n-propyl, isopropyl, n-butyl, isobutyl, tert-butyl, n-pentyl, isopentyl, n-hexyl, and isohexyl radicals.

7. Compound according to one of Claims 1 to 5, **characterized in that** the alkyl radicals having from 3 to 8 carbon atoms are chosen from the n-propyl, isopropyl, n-butyl, isobutyl, tert-butyl, n-pentyl, isopentyl, n-hexyl, isohexyl, n-heptyl, isoheptyl, n-octyl and isooctyl radicals.

8. Compound according to one of Claims 1 to 5, **characterized in that** the halogen atom is a fluorine, bromine or chlorine atom.

9. Compound according to one of Claims 1 to 5, **characterized in that** the alkoxy radical is a methoxy, ethoxy, isopropyloxy, tert-butoxy, hexyloxy, benzyloxy or phenoxy radical which can optionally be substituted by an alkyl radical having from 1 to 6 carbon atoms.

10. Compound according to one of Claims 1 to 5, **characterized in that** the polyether radical is a radical having from 1 to 6 carbon atoms which is interrupted by at least one oxygen atom, such as the methoxymethoxy, methoxymethylene, ethoxymethoxy, ethoxymethylene or methoxyethoxymethoxy radicals.

11. Compound according to one of Claims 1 to 5, **characterized in that** the alkyl ester radical is a carboxylate functional group substituted by an alkyl radical having from 1 to 6 carbon atoms.

12. Compound according to Claim 1, **characterized in that** it is taken, alone or as a mixture, from the group consisting of
1. 2(S)-Ethoxy-3-[3'-(3-heptyl-1-methylureido)-biphenyl-4-yl]propanoic acid,
2. 2(S)-Ethoxy-3-[3'-(1-methyl-3-pentylureido)-biphenyl-4-yl]propanoic acid,
3. 2(S)-Cyclopropylmethoxy-3-[3'-(3-heptyl-1-methylureido)biphenyl-4-yl]propanoic acid,
4. 2(S)-Propyloxy-3-[3'-(3-heptyl-1-methylureido)-biphenyl-4-yl]propanoic acid,
6. 2(S)-Allyloxy-3-[3'-(3-heptyl-1-methylureido)-biphenyl-4-yl]propanoic acid,
7. 2(S)-Cyclopropylmethoxy-3-[3'-(1-methyl-3-pentylureido)biphenyl-4-yl]propanoic acid,
8. 3-[3'-(1-Methyl-3-pentylureido)biphenyl-4-yl]-2(S)-propoxypropanoic acid,
9. 2(S)-Allyloxy-3-[3'-(1-methyl-3-pentylureido)biphenyl-4-yl]propanoic acid,
11. 2(S)-Methoxy-3-[3'-(1-methyl-3-pentylureido)-biphenyl-4-yl]propanoic acid,
12. 3-[3'-(3-Heptyl-1-methylureido)biphenyl-4-yl]-2(S)-methoxypropanoic acid,
13. 2(S)-Ethoxy-3-[3'-(1-methyl-3-propylureido)-biphenyl-4-yl]propanoic acid,
14. 3-[3'-(3-Cyclopropylmethyl-1-methylureido)-biphenyl-4-yl]-2(S)-ethoxypropanoic acid,
15. 3-[3'-(3-Cyclopentylmethyl-1-methylureido)-biphenyl-4-yl]-2(S)-ethoxypropanoic acid,
21. 2(S)-Cyclopropylmethoxy-3-[3'-(3-cyclopropylmethyl-l-methylureido)biphenyl-4-yl]propanoic acid,
22. 3-{3'-[3-(2-Cyclohexylethyl)-1-methylureido]-biphenyl-4-yl}-2(S)-ethoxypropanoic acid,
23. 2(R)-Ethoxy-3-[3'-(1-methyl-3-pentylureido)-biphenyl-4-yl]propanoic acid,
24. 2(R)-Ethoxy-3-[3'-(3-heptyl-1-methylureido)-biphenyl-4-yl]propanoic acid,
25. 2-Ethoxy-3-[3'-(3-heptyl-1-methylureido)biphenyl-4-yl]propanoic acid,
26. 2(R)-Allyloxy-3[3'-(3-heptyl-1-methylureido)-biphenyl-4-yl]propanoic acid,
27. 3-[3'-(3-Allyl-1-methylureido)biphenyl-4-yl]-2(S)-ethoxypropanoic acid.

13. Compound according to one of Claims 1 to 5, **characterized in that** it exhibits at least one of the following characteristics:
- R1 is chosen from an alkyl radical chosen from the methyl, ethyl, n-propyl, isopropyl, isobutyl or tert-butyl radicals, a cyclopropyl radical, or a methylcyclopropane group,
- R2 represents an alkyl radical chosen from the n-pentyl, isopentyl, n-hexyl, isohexyl, n-heptyl or isoheptyl radicals, or a cyclopentyl or cyclohexyl radical,
- R3 represents a hydrogen atom,
- R4 and R5 are a hydrogen atom.

14. Cosmetic composition, **characterized in that** it comprises, in a physiologically acceptable vehicle, at least one of the compounds as defined in any one of Claims 1 to 13.

15. Composition according to Claim 14, **characterized in that** the concentration of compound(s) according to one of Claims 1 to 13 is between 0.001% and 3% by weight, with respect to the total weight of the composition.

16. Cosmetic use of a composition as defined in either of Claims 14 and 15 for body or hair hygiene.

17. Compounds according to any one of Claims 1 to 13, as medicament.

18. Use of a compound according to any one of Claims 1 to 13 in the manufacture of a composition intended for the regulation and/or restoration of the metabolism of skin lipids.

19. Use of one or more compounds according to any one of Claims 1 to 13 in the manufacture of a pharmaceutical composition intended for the treatment:
1) of dermatological conditions linked to a disorder of keratinization involving differentiation and proliferation,
2) of other types of disorders of keratinization, 3) of other dermatological conditions with an inflammatory immunoallergic component, with or without cell proliferation disorder,
4) of all dermal or epidermal proliferations, whether they are benign or malignant and whether they are or are not of viral origin, the proliferations which can be induced by ultraviolet radiation,
5) of other dermatological disorders,
6) of dermatological or general conditions with an immunological component,
7) of skin disorders due to exposure to UV radiation, and also for repairing or combating skin ageing, whether photoinduced or chronologic or for reducing actinic keratoses and pigmentations or any pathology associated with chronologic or actinic ageing,
8) of disorders of the sebaceous function,
9) or the prevention of disorders of cicatrization or the prevention or the repair of stretch marks,
10) of disorders of pigmentation,
11) of conditions of the metabolism of lipids,
12) of inflammatory conditions,
13) or the prevention of cancerous or precancerous conditions,
14) or the prevention of alopecia of various origins, 15) of disorders of the immune system,
16) of conditions of the cardiovascular system.

20. Use according to claim 19 **characterized in that**:
1) the dermatological conditions linked to a disorder of keratinization involving differentiation and proliferation is chosen from acne vulgaris, comedonic or polymorphic acne, acne rosacea, nodulocystic acne, acne conglobata, senile acne, or solar, drug or occupational acne,
2) the other types of disorders of keratinisation are chosen from ichthyoses, ichthyosiform conditions, Darier's disease, palmoplantar keratoderma, leucoplakia and leucoplakiform conditions or cutaneous or mucosal (oral) lichen,
3) the other dermatological conditions with an inflammatory immunoallergic component, with or without cell proliferation disorder, are chosen from all forms of psoriasis, whether cutaneous, mucosal or ungual, and even psoriatic rheumatism, or alternatively eczema, or respiratory atopy or alternatively gingival hypertrophy,
4) the dermal or epidermal proliferations, whether they are benign or malignant and whether they are or are not of viral origin are chosen from common warts, flat warts and epidermodysplasia verruciformis, florid or oral papillomatoses, T lymphoma, basal cell and prickle cell epithelioma, or keratoacanthomas,
5) the other dermatological disorders are chosen from immune dermatoses, lupus erythematosus, immune bullous diseases and collagen diseases, or scleroderma,
7) the skin disorders due to exposure to UV radiation, and also for repairing or combating skin ageing, is xerosis,
8) the disorders of the sebaceous function, are chosen from hyperseborrhoea of acne or simple seborrhoea,
10) the disorders of pigmentation are chosen from hyperpigmentation, melasma, hypopigmentation or vitiligo,
11) the conditions of the metabolism of lipids are chosen from obesity, hyperlipidaemia or non-insulin-dependent diabetes,
12) the inflammatory conditions is arthritis,
14) the prevention of alopecia of various origins is alopecia due to chemotherapy or to radiation,
15) the disorders of the immune system are chosen from asthma, type I diabetes mellitus, multiple sclerosis or other selective dysfunctions of the immune system,
16) the condition of the cardiovascular system is arteriosclerosis or hypertension.

21. Pharmaceutical composition, **characterized in that** it comprises, in a physiologically acceptable vehicle, at least one of the compounds as defined in any one of Claims 1 to 13.

22. Composition according to Claim 21, **characterized in that** the concentration of compound(s) according to one of Claims 1 to 13 is between 0.001% and 10% by weight, with respect to the total weight of the composition.

23. Composition according to Claim 22, **characterized in that** the concentration of compound(s) according to one of Claims 1 to 13 is between 0.01% and 1% by weight, with respect to the total weight of the composition.

24. Process for the synthesis of the compounds of formula (I) or of the possible isomers, optical and/or geometrical, pure or as a mixture, in all proportions, of the said compounds of formula (I), of the possible tautomeric forms, or of the salts of the said compounds of formula (I), **characterized in that** it comprises the following stages:
a) Preparation of the compound of formula 1: 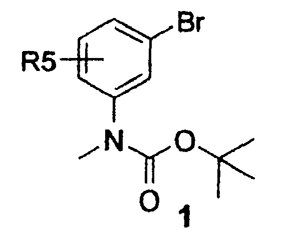 where R5 represents a hydrogen atom,
from 3-bromoaniline, by protecting the amine with di(tert-butyl) dicarbonate and by then carrying out a methylation in the presence of sodium hydride;
b) Preparation of compound 2: 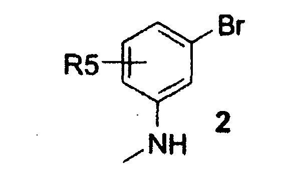 with R5 as defined above,
by treating compound 1 with an acid;
c) Preparation of compound 3: 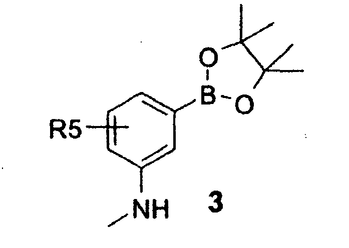 with R5 as defined above,
by the reaction of compound 2 with pinacolborane in the presence of a catalyst;
d) Preparation of compound 5: 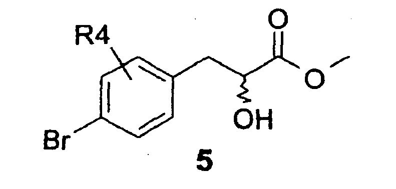 with R4 representing a hydrogen atom,
by treating the epoxide 4 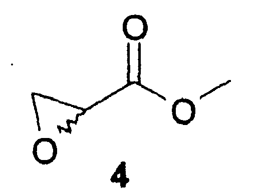 with an aryl cuprate;
e) Preparation of compound 6: 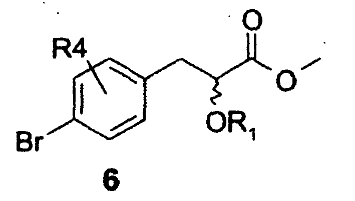 with R1 representing an alkyl group having from 1 to 6 carbon atoms chosen from the methyl, ethyl, n-propyl, isopropyl, isobutyl, tert-butyl, n-pentyl, isopentyl, n-hexyl, and isohexyl radicals; a cyclopropyl, a cyclohexyl, an ethylenyl, an allyl, a propenyl, a butenyl, a pentenyl or an hexenyl radical, an acetyl group, a methylcyclopropane group, an aralkyl group or an aryl group, the aryl group being chosen from a biphenyl, cinnamyl or naphthyl radical which can be mono- or disubstituted by a halogen atom, a CF₃ radical, an alkyl radical having from 1 to 6 carbon atoms, an alkoxy radical having from 1 to 6 carbon atoms, a nitro functional group, a polyether radical, an aryl radical, a benzoyl radical, an alkyl ester group, a carboxylic acid, a hydroxyl radical optionally protected by an acetyl or benzoyl group, or an amino functional group optionally protected by an acetyl or benzoyl group or optionally substituted by at least one alkyl having from 1 to 6 carbon atoms, and
the aralkyl group being chosen from a phenethyl or naphth-2-ylmethyl radical which is unsubstituted or substituted by one or more radicals chosen from a halogen atom, a CF₃ radical, an alkyl radical having from 1 to 6 carbon atoms, an alkoxy radical having from 1 to 6 carbon atoms, a hydroxyl radical or an amino functional group which is unprotected or unsubstituted or optionally substituted by at least one alkyl having from 1 to 6 carbon atoms, or a carboxyl functional group,
and R4 as defined above;
by reacting compound 5 with an alkyl halide in the presence of silver oxide;
f) Preparation of compound 7: 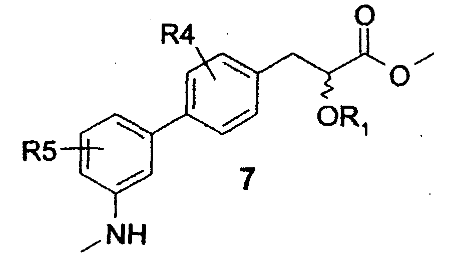 with R1, R4 and R5 as defined above,
by the coupling of compound 6 and compound 3 according to a reaction of Suzuki type in the presence of tetrakis(triphenylphosphine)palladium;
g) Preparation of compound 8: 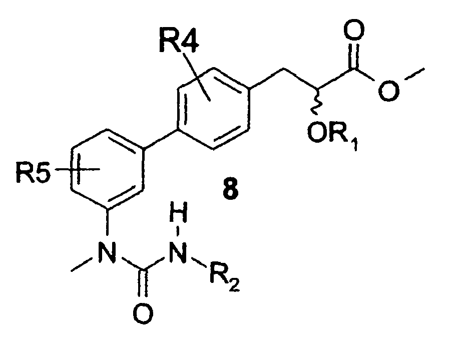 with R2 representing an alkyl group having from 3 to 8 carbon atoms, or a cyclopropyl, a cyclopentyl, a cyclohexyl, an allyl, a propenyl, a butenyl, a pentenyl, a hexenyl, a heptenyl or an octenyl radical, and R1, R4 and R5 as defined above,
by the reaction between compound 7 and an alkyl isocyanate;
h) Preparation of compound (I) 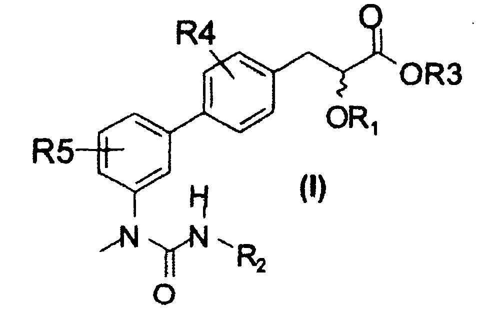
with R3 is a hydrogen atom: by saponification of compound 8 in the presence of a base.

25. Process for the synthesis of the compounds of formula (I) or of the possible isomers, optical and/or geometrical, pure or as a mixture, in all proportions, of the said compounds of formula (I), of the possible tautomeric forms, or of the salts of the said compounds of formula (I), **characterized in that** it comprises the following stages:
a) Preparation of the compound of formula 1: 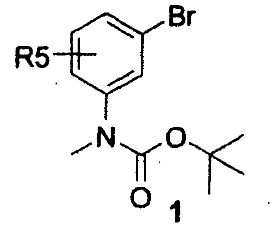 where R5 represents a hydrogen atom,
from 3-bromoaniline, by protecting the amine with di(tert-butyl) dicarbonate and by then carrying out a methylation in the presence of sodium hydride;
b) Preparation of compound 2: 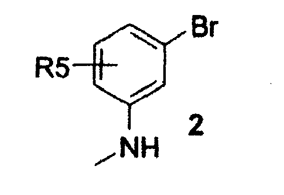 with R5 as defined above,
by treating compound 1 with an acid;
c) Preparation of compound 3: 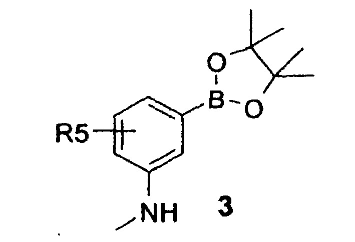 with R5 as defined above,
by the reaction of compound 2 with pinacolborane in the presence of a catalyst;
d) Preparation of compound 5: 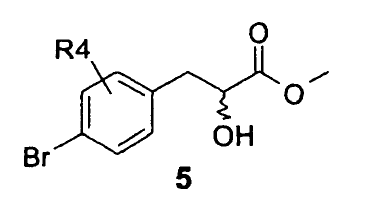 with R4 representing a hydrogen atom,
by treating the epoxide 4 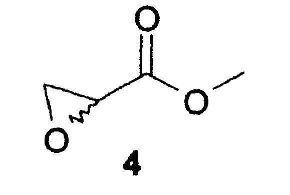 with an aryl halide in the presence of tert-butyllithium and of copper cyanide;
i) Preparation of compound 10: 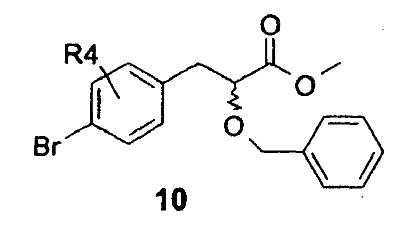 with R4 as defined above,
by treating compound 5 with a benzyl halide in the presence of silver oxide;
j) Preparation of compound 11: 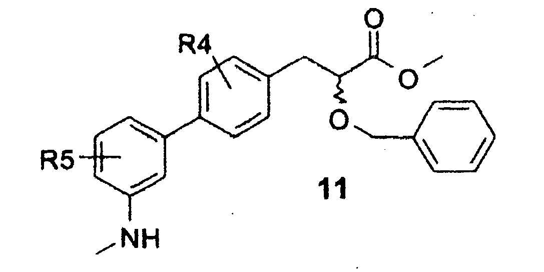 with R4 and R5 as defined above,
by coupling compound 10 with compound 3 by a reaction of Suzuki type using a palladium catalyst;
k) Preparation of compound 12, in which R2, R4 and R5 are as defined in the formula (I), 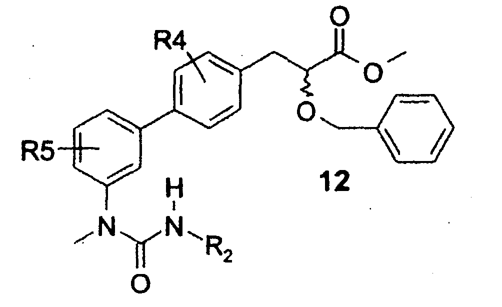 with R2 representing an alkyl group having from 3 to 8 carbon atoms, or a cyclopropyl, a cyclopentyl, a cyclohexyl, an allyl, a propenyl, a butenyl, a pentenyl, a hexenyl, a heptenyl or an octenyl radical, and R4 and R5 as defined above,
by the reaction between compound 11 and an alkyl isocyanate;
l) Preparation of the alcohol 13: 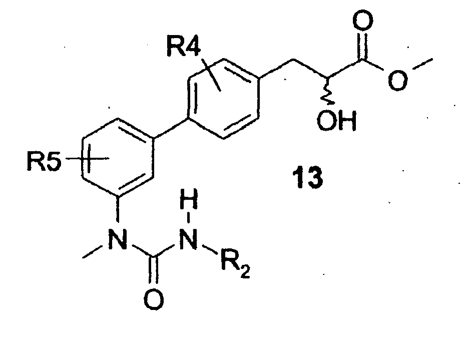 with R2, R4 and R5 as defined above,
by hydrogenolysis of compound 12 with hydrogen in the presence of palladium-on-charcoal;
m) Preparation of compound 8, in which R1, R2, R4 and R5 are as defined in the formula (I), 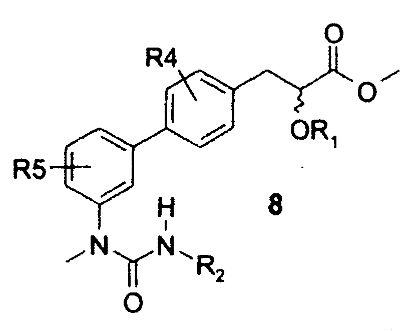 with R1 representing an alkyl group having from 1 to 6 carbon atoms chosen from the methyl, ethyl, n-propyl, isopropyl, isobutyl, tert-butyl, n-pentyl, isopentyl, n-hexyl, and isohexyl radicals; an acetyl group, a methylcyclopropane group, an aralkyl group or an aryl group,
the aryl group being chosen from a biphenyl, cinnamyl or naphthyl radical which can be mono- or disubstituted by a halogen atom, a CF₃ radical, an alkyl radical having from 1 to 6 carbon atoms, an alkoxy radical having from 1 to 6 carbon atoms, a nitro functional group, a polyether radical, an aryl radical, a benzoyl radical, an alkyl ester group, a carboxylic acid, a hydroxyl radical optionally protected by an acetyl or benzoyl group, or an amino functional group optionally protected by an acetyl or benzoyl group or optionally substituted by at least one alkyl having from 1 to 6 carbon atoms, and
the aralkyl group being chosen from a phenethyl or naphth-2-ylmethyl radical which is unsubstituted or substituted by one or more radicals chosen from a halogen atom, a CF₃ radical, an alkyl radical having from 1 to 6 carbon atoms, an alkoxy radical having from 1 to 6 carbon atoms, a hydroxyl radical or an amino functional group which is unprotected or unsubstituted or optionally substituted by at least one alkyl having from 1 to 6 carbon atoms, or a carboxyl functional group;
and R2, R4 and R5 as defined above;
by reacting compound 13 with an alkyl halide in the presence of silver oxide;
n) Preparation of compound (I): 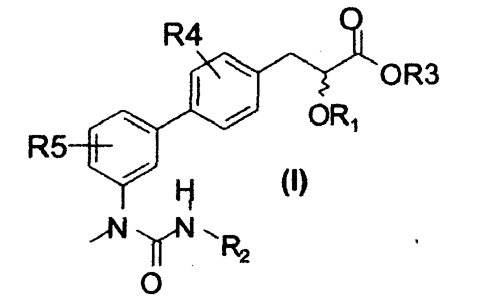
with R3 is a hydrogen atom: by saponification of compound 8 in the presence of a base.

## Patentansprüche

1. Verbindung der Formel (I): 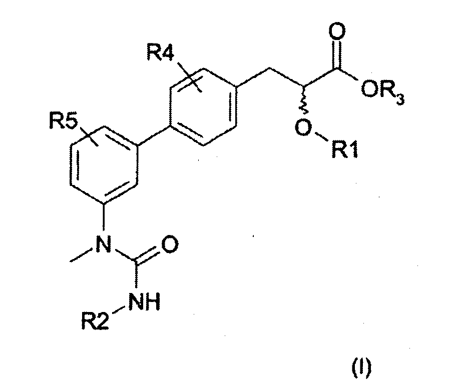 in der Formel:
- R₁ bedeutet eine Alkylgruppe mit 1 bis 6 Kohlenstoffatomen, die unter Methyl, Ethyl, n-Propyl, Isopropyl, Isobutyl, tert-Butyl, n-Pentyl, Isopentyl, n-Hexyl und Isohexyl ausgewählt ist; eine Cyclopropylgruppe, eine Cyclohexylgruppe, eine Ethylenylgruppe, eine Allylgruppe, eine Propenylgruppe, eine Butenylgruppe, eine Pentenylgruppe oder eine Hexenylgruppe, eine Acetylgruppe, eine Methylcyclopropangruppe, eine Aralkylgruppe oder eine Arylgruppe, wobei die Arylgruppe unter einer Biphenyl-, Cinnamyl- oder Naphthylgruppe ausgewählt ist, die ein- oder zweifach substituiert sein kann mit einem Halogenatom, einer Gruppe CF₃, einer Alkylgruppe mit 1 bis 6 Kohlenstoffatomen, einer Alkoxygruppe mit 1 bis 6 Kohlenstoffatomen, einer Nitrofunktion, einer Polyethergruppe, einer Arylgruppe, einer Benzoylgruppe, einer Alkylestergruppe, einer Carbonsäurefunktion, einer Hydroxygruppe, die gegebenenfalls mit einer Acetylgruppe oder Benzoylgruppe geschützt ist, oder einer Aminofunktion, die gegebenenfalls mit einer Acetylgruppe oder Benzoylgruppe geschützt oder gegebenenfalls mit mindestens einer Alkylgruppe mit 1 bis 6 Kohlenstoffatomen substituiert ist, und wobei die Aralkylgruppe unter Phenethyl oder Naphth-2-ylmethyl ausgewählt ist, die unsubstituiert vorliegen oder mit einer oder mehr Gruppen substituiert sind, die ausgewählt sind unter einem Halogenatom, einer Gruppe CF₃, einer Alkylgruppe mit 1 bis 6 Kohlenstoffatomen, einer Alkoxygruppe mit 1 bis 6 Kohlenstoffatomen, einer Hydroxygruppe oder einer Aminofunktion, die ungeschützt oder unsubstituiert oder gegebenenfalls mit mindestens einer Alkylgruppe mit 1 bis 6 Kohlenstoffatomen substituiert ist, oder einer Carboxyfunktion,
- R₂ bedeutet eine Alkylgruppe mit 3 bis 8 Kohlenstoffatomen oder Cyclopropyl, Cyclopentyl, Cyclohexyl, Allyl, Propenyl, Butenyl, Pentenyl, Hexenyl, Heptenyl oder Octenyl;
- R₃ ist ein Wasserstoffatom;
- R₄ und R₅ bedeuten ein Wasserstoffatom;
und die möglichen optischen und/ oder geometrischen Isomere der Verbindung der Formel (I) in reiner Form oder als Gemisch in beliebigen Anteilen und die möglichen tautomeren Formen und ferner die Salze der Verbindung der Formel (I).

2. Verbindung nach Anspruch 1, **dadurch gekennzeichnet, dass** sie in der Form von Alkalimetallsalzen oder Erdalkalimetallsalzen oder Salzen eines organischen Amins vorliegt.

3. Verbindung nach Anspruch 2, **dadurch gekennzeichnet, dass** sie in der Form des Natriumsalzes vorliegt.

4. Verbindung nach Anspruch 2, **dadurch gekennzeichnet, dass** sie in der Form eines Aminosäuresalzes vorliegt.

5. Verbindung nach Anspruch 4, **dadurch gekennzeichnet, dass** sie in der Form eines Argininsalzes oder Lysinsalzes vorliegt.

6. Verbindung nach einem der Ansprüche 1 bis 5, **dadurch gekennzeichnet, dass** die Alkylgruppen mit 1 bis 6 Kohlenstoffatomen unter den Gruppen Methyl, Ethyl, n-Propyl, Isopropyl, n-Butyl, Isobutyl, *tert*-Butyl, n-Pentyl, Isopentyl, n-Hexyl und Isohexyl ausgewählt sind.

7. Verbindung nach einem der Ansprüche 1 bis 5, **dadurch gekennzeichnet, dass** die Alkylgruppen mit 3 bis 8 Kohlenstoffatomen unter den Gruppen n-Propyl, Isopropyl, n-Butyl, Isobutyl, *tert*-Butyl, n-Pentyl, Isopentyl, n-Hexyl, Isohexyl, n-Heptyl, Isoheptyl, n-Octyl und Isooctyl ausgewählt sind.

8. Verbindung nach einem der Ansprüche 1 bis 5, **dadurch gekennzeichnet, dass** das Halogenatom unter Fluor, Brom oder Chlor ausgewählt ist.

9. Verbindung nach einem der Ansprüche 1 bis 5, **dadurch gekennzeichnet, dass** die Alkoxygruppe eine Methoxygruppe, Ethoxygruppe, Isopropyloxygruppe, tert-Butoxygruppe, Hexyloxygruppe, Benzyloxygruppe oder Phenoxygruppe ist, die gegebenenfalls mit einer Alkylgruppe mit 1 bis 6 Kohlenstoffatomen substituiert sind.

10. Verbindung nach einem der Ansprüche 1 bis 5, **dadurch gekennzeichnet, dass** die Polyethergruppe eine Gruppe mit 1 bis 6 Kohlenstoffatomen ist, die durch mindestens ein Sauerstoffatom unterbrochen ist, wie Methoxymethoxy, Methoxymethylen, Ethoxymethoxy, Ethoxymethylen oder Methoxyethoxymethoxy.

11. Verbindung nach einem der Ansprüche 1 bis 5, **dadurch gekennzeichnet, dass** die Alkylestergruppe eine Carboxylatfunktion ist, die mit einer Alkylgruppe mit 1 bis 6 Kohlenstoffatomen substituiert ist.

12. Verbindung nach Anspruch 1, **dadurch gekennzeichnet, dass** sie einzeln oder als Gemisch unter den folgenden Verbindungen ausgewählt ist:
1. 2(S)-Ethoxy-3-[3'-(3-heptyl-1-methylureido)-biphenyl-4-yl]-propansäure,
2. 2(S)-Ethoxy-3-[3'-(1-methyl-3-pentylureido)-biphenyl-4-yl]-propansäure,
3. 2(S)-Cyclopropylmethoxy-3-[3'-(3-heptyl-1-methyl-ureido)-biphenyl-4-yl]-propansäure,
4. 2(S)-Propyloxy-3-[3'-(3-heptyl-1-methylureido)-biphenyl-4-yl]-propansäure,
6. 2(S)-Allyloxy-3-[3'-(3-heptyl-1-methylureido)-biphenyl-4-yl]-propansäure,
7. 2(S)-Cyclopropylmethoxy-3-[3'-(1-methyl-3-pentyl-ureido) biphenyl-4-yl]-propansäure,
8. 3-[3'-(1-Methyl-3-pentylureido)biphenyl-4-yl]-2(S)-propoxy-propansäure,
9. 2(S)-Allyloxy-3-[3'-(1-methyl-3-pentylureido)-biphenyl-4-yl]-propansäure,
11. 2(S)-Methoxy-3-[3'-(1-methyl-3-pentylureido)-biphenyl-4-yl]-propansäure,
12. 3-[3'-(3-Heptyl-1-methylureido)biphenyl-4-yl]-2(S)-methoxy-propansäure,
13. 2(S)-Ethoxy-3-[3'-(1-methyl-3-propylureido)-biphenyl-4-yl]-propansäure,
14. 3-[3'-(3-Cyclopropylmethyl-1-methylureido)-biphenyl-4-yl]-2 (S)-ethoxy-propansäure,,
15. 3-[3'-(3-Cyclopentylmethyl-1-methylureido)-biphenyl-4-yl]-2 (S)-ethoxy-propansäure,
21. 2(S)-Cyclopropylmethoxy-3-[3'-(3-cyclopropyl-methyl-1-methylureido)-biphenyl-4-yl]-propansäure,
22. 3-{3'-[3-(2-Cyclohexylethyl)-1-methylureido]-biphenyl-4-yl}-2 (S)-ethoxy-propansäure,
23. 2(R)-Ethoxy-3-[3'-(1-methyl-3-pentylureido)-biphenyl-4-yl]-propansäure,
24. 2(R)-Ethoxy-3-[3'-(3-heptyl-1-methylureido)-biphenyl-4-yl]-propansäure,
25. 2-Ethoxy-3-[3'-(3-heptyl-1-methylureido)-biphenyl-4-yl]-propansäure,
26. 2(R)-Allyloxy-3[3'-(3-heptyl-1-methylureido)-biphenyl-4-yl]-propansäure,
27. 3-[3'-(3-Allyl-1-methylureido)-biphenyl-4-yl]-2(S)-ethoxy-propansäure.

13. Verbindung nach einem der Ansprüche 1 bis 5, **dadurch gekennzeichnet, dass** sie mindestens eine der folgenden Eigenschaften aufweist:
- R₁ ist eine Alkylgruppe, die unter Methyl, Ethyl, n-Propyl, Isopropyl, Isobutyl oder *tert*-Butyl, Cyclopropyl oder Methylcyclopropan ausgewählt ist,
- R₂ ist eine Alkylgruppe, die unter n-Pentyl, Isopentyl, n-Hexyl, Isohexyl, n-Heptyl oder Isoheptyl, Cyclopentyl oder Cyclohexyl ausgewählt ist,
- R₃ bedeutet ein Wasserstoffatom,
- R₄ und R₅ bedeuten Wasserstoffatome.

14. Kosmetische Zusammensetzung, **dadurch gekennzeichnet, dass** sie in einem physiologisch akzeptablen Träger mindestens eine Verbindung enthält, wie sie in einem der Ansprüche 1 bis 13 definiert ist.

15. Zusammensetzung nach Anspruch 14, **dadurch gekennzeichnet, dass** die Konzentration der Verbindung(en) nach einem der Ansprüche 1 bis 13 im Bereich von 0,001 bis 3 Gew.-%, bezogen auf das Gesamtgewicht der Zusammensetzung, liegt.

16. Kosmetische Verwendung einer Zusammensetzung nach einem der Ansprüche 14 und 15 für die Körper- oder Haarpflege.

17. Verbindungen nach einem der Ansprüche 1 bis 13 als Arzneimittel.

18. Verwendung einer Verbindung nach einem der Ansprüche 1 bis 13 für die Herstellung einer Verbindung, die dazu vorgesehen ist, den Metabolismus der Hautlipide zu regulieren und/oder wiederherzustellen.

19. Verwendung einer oder mehrerer Verbindungen nach einem der Ansprüche 1 bis 13 zur Herstellung einer pharmazeutischen Zusammensetzung, die vorgesehen ist für die Behandlung von:
1) dermatologischen Erkrankungen, die mit einer Verhornungsstörung verbunden sind, die auf der Differenzierung und Proliferation beruht;
2) anderen Arten von Keratinisierungsstörungen;
3) anderen dermatologischen Erkrankungen mit entzündlicher immunoallergischer Komponente, mit oder ohne einer Störung der Proliferation der Zellen;
4) allen Proliferationen der Dermis oder Epidermis, die gutartig oder bösartig und gegebenenfalls viralen Ursprungs sein können, Proliferationen, die von UV-Strahlung induziert sein können;
5) anderen dermatologischen Störungen;
6) dermatologischen oder allgemeinen Erkrankungen mit immunologischer Komponente;
7) Hautstörungen, die mit einer UV-Exposition zusammenhängen; und ferner zur Behebung oder zur Bekämpfung der altersbedingten oder lichtinduzierten Hautalterung, oder zur Verminderung aktinischer Keratosen und Pigmentierungen; oder allen Pathologien, die mit der altersbedingten oder aktinischen Hautalterung einhergehen;
8) Störungen der Talgdrüsenfunktion;
9) Vorbeugung von Störungen der Wundheilung oder Vorbeugung oder Behebung von Streifen;
10) Pigmentierungsstörungen;
11) Störungen des Fettstoffwechsels;
12) Entzündungen;
13) Vorbeugung von kanzerösen oder präkanzerösen Zuständen;
14) Vorbeugung von Alopezie unterschiedlicher Herkunft;
15) Erkrankungen des Immunsystems; oder
16) Störungen des kardiovaskulären Systems.

20. Verwendung nach Anspruch 19, **dadurch gekennzeichnet, dass**
1) die dermatologischen Erkrankungen, die mit einer Keratinisierungsstörung zusammenhängen, die auf der Differenzierung und Proliferation beruht, unter Acne vulgaris, Acne comedonica, polymorpher Akne, Acne rosacea, nodulocystischer Akne, Acne conglobata, Acne senilis, Acne solaris, Acne medicamentosa oder Acne professionalis ausgewählt sind;
2) die anderen Arten von Keratinisierungsstörungen unter Ichthyosis, ichtyosisartigen Zuständen, der Darier Krankheit, Palmoplantarkeratosen, Leukoplakien und leucoplakieformen Zuständen oder Lichen der Haut oder der Schleimhäute (oral) ausgewählt sind;
3) dermatologischen Erkrankungen mit immunoallergischer entzündlicher Komponente mit oder ohne Störung der Zellproliferation unter Psoriasis der Haut, der Schleimhäute oder der Nägel oder sogar Psoriasis arthropathica, oder alternativ Ekzemen, Atopie der Atemwege oder Hypertrophie des Zahnfleisches ausgewählt sind;
4) die Proliferationen der Dermis oder Epidermis, die gutartig oder bösartig und gegebenenfalls viralen Ursprungs sein können, unter Verrucae vulgares, Verrucae planae und Epidermodysplasia verruciformis, Papillomatosis oralis oder florida, T-Lymphom, Epithelioma basocellulare und spinocellulare oder Keratoacanthomen ausgewählt sind;
5) die weiteren dermatologischen Störungen unter Immundermatosen, Lupus erythematodes, bullösen Immunerkrankungen, Kollagenerkrankungen oder Sklerodermie ausgewählt sind;
7) es sich bei den Hautstörungen, die mit einer UV-Exposition zusammenhängen, und ferner zur Behebung oder zur Bekämpfung der Hautalterung, um Xerosis handelt;
8) die Störungen der Talgdrüsenfunktion unter Hyperseborrhoe bei Akne oder Seborrhoe simplex ausgewählt sind;
10) die Pigmentierungsstörungen unter Hyperpigmentierung, Melasmen, Hypopigmentierung oder Vitiligo ausgewählt sind;
11) die Fettstoffwechselstörung unter Adipositas, Hyperlipidämie oder nicht-insulinpflichtigem Diabetes ausgewählt ist;
12) es sich bei den Entzündungen um Arthritis handelt;
14) sich die Vorbeugung von Alopezie unterschiedlicher Herkunft auf durch Chemotherapie oder Strahlung verursachte Alopezie bezieht;
15) die Immunerkrankungen unter Asthma, Diabetes vom Typ 1, Multipler Sklerose und anderen selektiven Funktionsstörungen des Immunsystems ausgewählt sind;
16) es sich bei der Erkrankung des kardiovaskulären Systems um Arteriosklerose oder Bluthochdruck handelt.

21. Pharmazeutische Zusammensetzung, **dadurch gekennzeichnet, dass** sie in einem physiologisch akzeptablen Trägerstoff mindestens eine Verbindung nach einem der Ansprüche 1 bis 13 enthält.

22. Zusammensetzung nach Anspruch 21, **dadurch gekennzeichnet, dass** die Konzentration der Verbindung(en) nach einem der Ansprüche 1 bis 13 im Bereich von 0,001 bis 10 Gew.-%, bezogen auf das Gesamtgewicht der Zusammensetzung, liegt.

23. Zusammensetzung nach Anspruch 22, **dadurch gekennzeichnet, dass** die Konzentration der Verbindung(en) nach einem der Ansprüche 1 bis 13 im Bereich von 0,01 bis 1 Gew.-%, bezogen auf das Gesamtgewicht der Zusammensetzung, liegt.

24. Verfahren zur Synthese von Verbindungen der Formel (I) oder der möglichen optischen und/oder geometrischen Isomere der Verbindungen der Formel (I) in reiner Form oder als Gemisch in allen Mengenanteilen, der möglichen tautomeren Formel oder der Salze der Verbindungen der Formel (I), **dadurch gekennzeichnet, dass** es die folgenden Schritte umfasst:
a) Herstellung der Verbindung der Formel 1: 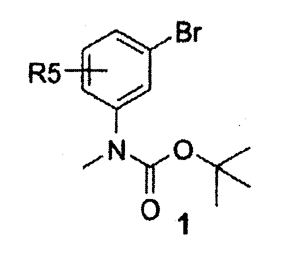 wobei R₅ ein Wasserstoffatom bedeutet,
ausgehend von 3-Bromanilin, indem das Amin mit Di(tert-butyl)dicarbonat geschützt wird und anschließend eine Methylierung in Gegenwart von Natriumhydrid durchgeführt wird;
b) Herstellung der Verbindung 2: 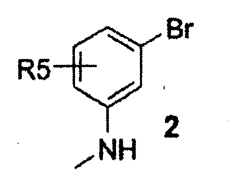 wobei R₅ die oben angegebene Bedeutung hat;
indem die Verbindung der Formel 1 mit einer Säure behandelt wird;
c) Herstellung der Verbindung 3: 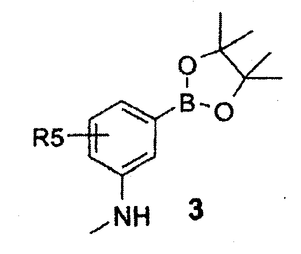 wobei R₅ die oben angegebene Bedeutung hat;
durch Umsetzung der Verbindung 2 mit Pinacolboran in Gegenwart eines Katalysators;
d) Herstellung der Verbindung 5: 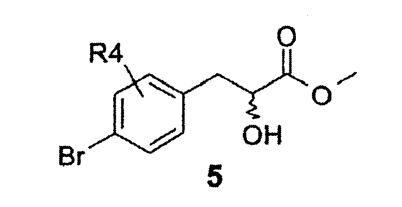 wobei R₄ ein Wasserstoffatom bedeutet;
indem das Epoxid 4 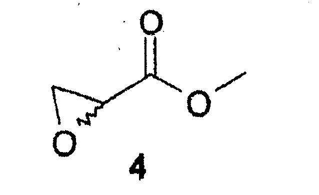 mit einem Arylcuprat umgesetzt wird;
e) Herstellung der Verbindung 6: 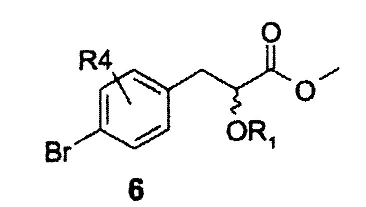 wobei R₁ bedeutet: eine Alkylgruppe mit 1 bis 6 Kohlenstoffatomen, die unter Methyl, Ethyl, n-Propyl, Isopropyl, Isobutyl, tert-Butyl, n-Pentyl, Isopentyl, n-Hexyl und Isohexyl ausgewählt ist; eine Cyclopropylgruppe, eine Cyclohexylgruppe, eine Ethylenylgruppe, eine Allylgruppe, eine Propenylgruppe, eine Butenylgruppe, eine Pentenylgruppe oder eine Hexenylgruppe, eine Acetylgruppe, eine Methylcyclopropangruppe, eine Aralkylgruppe oder eine Arylgruppe,
wobei die Arylgruppe unter einer Biphenyl-, Cinnamyl- oder Naphthylgruppe ausgewählt ist, die ein- oder zweifach substituiert sein kann mit einem Halogenatom, einer Gruppe CF₃, einer Alkylgruppe mit 1 bis 6 Kohlenstoffatomen, einer Alkoxygruppe mit 1 bis 6 Kohlenstoffatomen, einer Nitrofunktion, einer Polyethergruppe, einer Arylgruppe, einer Benzoylgruppe, einer Alkylestergruppe, einer Carbonsäurefunktion, einer Hydroxygruppe, die gegebenenfalls mit einer Acetylgruppe oder Benzoylgruppe geschützt ist, oder einer Aminofunktion, die gegebenenfalls mit einer Acetylgruppe oder Benzoylgruppe geschützt oder gegebenenfalls mit mindestens einer Alkylgruppe mit 1 bis 6 Kohlenstoffatomen substituiert ist, und
wobei die Aralkylgruppe unter Phenethyl oder Naphth-2-ylmethyl ausgewählt ist, die unsubstituiert vorliegen oder mit einer oder mehr Gruppen substituiert sind, die ausgewählt sind unter einem Halogenatom, einer Gruppe CF₃, einer Alkylgruppe mit 1 bis 6 Kohlenstoffatomen, einer Alkoxygruppe mit 1 bis 6 Kohlenstoffatomen, einer Hydroxygruppe oder einer Aminofunktion, die ungeschützt oder unsubstituiert oder gegebenenfalls mit mindestens einer Alkylgruppe mit 1 bis 6 Kohlenstoffatomen substituiert ist, oder einer Carboxyfunktion, und wobei R₄ die oben angegebenen Bedeutungen hat;
indem Verbindung 5 in Gegenwart von Silberoxid mit einem Alkylhalid umgesetzt wird;
f) Herstellung der Verbindung 7: 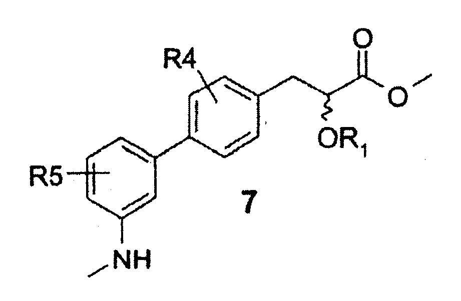 wobei die Gruppen R₁, R₄ und R₅ die oben angegebenen Bedeutungen aufweisen,
indem die Verbindung 6 und die Verbindung 3 nach einer Reaktion vom Suzuki-Typ in Gegenwart von Tetrakis(triphenylphosphin)palladium gekuppelt werden;
g) Herstellung der Verbindung 8: 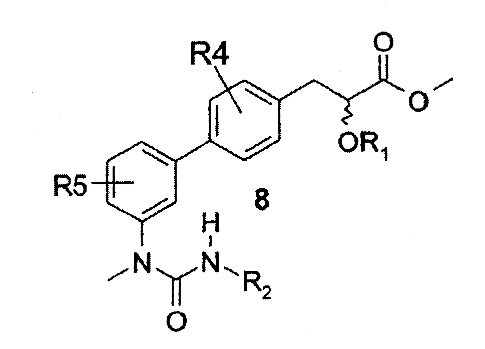 wobei R₂ eine Alkylgruppe mit 3 bis 8 Kohlenstoffatomen oder Cyclopropyl, Cyclopentyl, Cyclohexyl, Allyl, Propenyl, Butenyl, Pentenyl, Hexenyl, Heptenyl oder Octenyl bedeutet,
und R₁, R₄ und R₅ die oben angegebenen Bedeutungen aufweisen,
indem die Verbindung 7 mit einem Alkylisocyanat umgesetzt wird,
h) Herstellung der Verbindung (I): 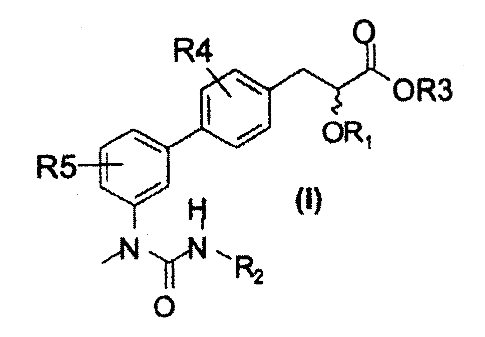
wobei R₃ ein Wasserstoffatom bedeutet,
durch Verseifung der Verbindung 8 in Gegenwart einer Base.

25. Verfahren zur Synthese von Verbindungen der Formel (I) oder der möglichen optischen und/oder geometrischen Isomere der Verbindungen der Formel (I) in reiner Form oder als Gemisch in allen Mengenanteilen, der möglichen tautomeren Formel oder der Salze der Verbindungen der Formel (I), **dadurch gekennzeichnet, dass** es die folgenden Schritte umfasst:
a) Herstellung der Verbindung der Formel 1: 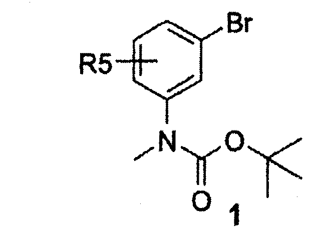 wobei R₅ ein Wasserstoffatom bedeutet,
ausgehend von 3-Bromanilin, indem das Amin mit Di(tert-butyl)-dicarbonat geschützt wird und anschließend eine Methylierung in Gegenwart von Natriumhydrid durchgeführt wird;
b) Herstellung der Verbindung 2: 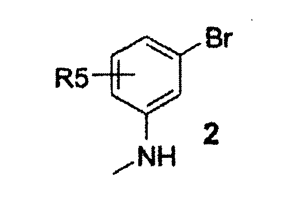 wobei R₅ die oben angegebene Bedeutung hat;
indem die Verbindung 1 mit einer Säure behandelt wird;
c) Herstellung der Verbindung 3: 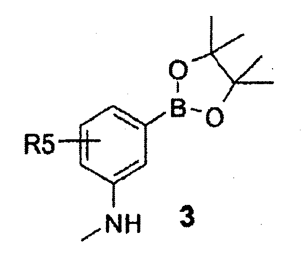 wobei R₅ die oben angegebene Bedeutung hat;
durch Umsetzung der Verbindung 2 mit Pinacolboran in Gegenwart eines Katalysators;
d) Herstellung der Verbindung 5: 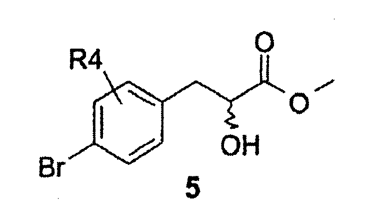 wobei R₄ ein Wasserstoffatom bedeutet;
indem das Epoxid 4 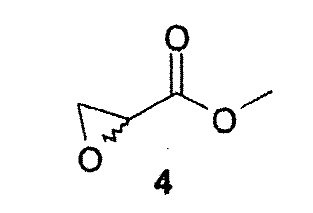 in Gegenwart von tert-Butyllithium und Kupfercyanid mit einem Arylhalid umgesetzt wird;
i) Herstellung der Verbindung 10: 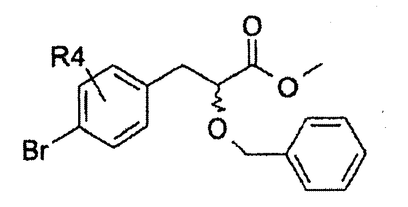 wobei R₄ die oben angegebenen Bedeutungen hat,
indem die Verbindung 5 mit einem Benzylhalid in Gegenwart von Silberoxid umgesetzt wird;
j) Herstellung der Verbindung 11: 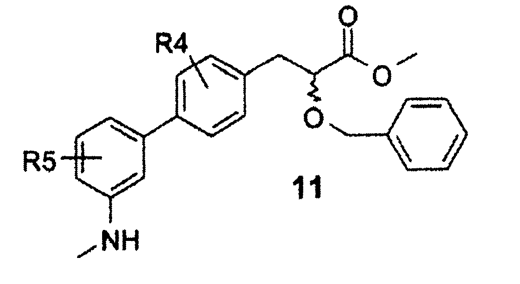 wobei die Gruppen R₄ und R₅ die oben angegebenen Bedeutungen aufweisen,
indem die Verbindung 10 und die Verbindung 3 nach einer Reaktion vom Suzuki-Typ unter Verwendung eines Palladiumkatalysators gekuppelt werden;
k) Herstellung der Verbindung 12, wobei die Gruppen R₂, R₄ und R₅ die für Formel (I) angegebenen Bedeutungen aufweisen: 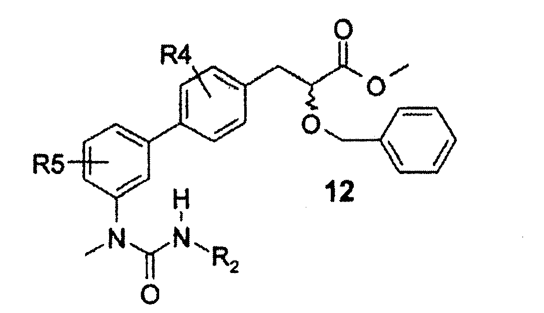 wobei R₂ eine Alkylgruppe mit 3 bis 8 Kohlenstoffatomen oder Cyclopropyl, Cyclopentyl, Cyclohexyl, Allyl, Propenyl, Butenyl, Pentenyl, Hexenyl, Heptenyl oder Octenyl bedeutet,
und R₄ und R₅ die oben angegebenen Bedeutungen aufweisen, indem die Verbindung 11 mit einem Alkylisocyanat umgesetzt wird;
l) Herstellung des Alkohols 13: 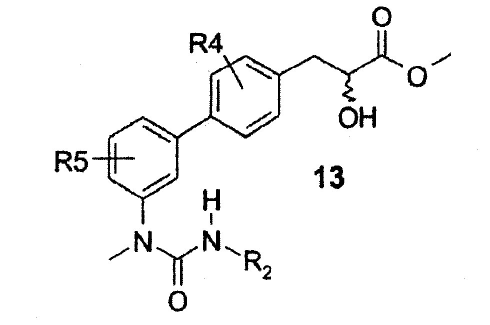 wobei die Gruppen R₂, R₄ und R₅ die oben angegebenen Bedeutungen aufweisen,
durch Hydrogenolyse der Verbindung 12 mit Wasserstoff in Gegenwart von Palladium auf Kohle;
m) Herstellung der Verbindung 8, wobei die Gruppen R₁, R₂, R₄ und R₅ die oben in Formel (I) angegebenen Bedeutungen aufweisen: 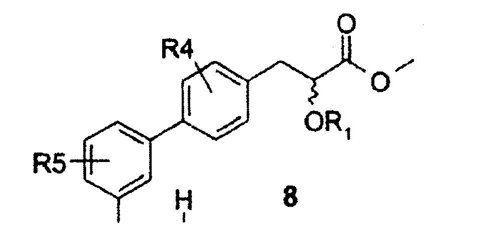 wobei R₁ bedeutet: eine Alkylgruppe mit 1 bis 6 Kohlenstoffatomen, die unter Methyl, Ethyl, n-Propyl, Isopropyl, Isobutyl, tert-Butyl, n-Pentyl, Isopentyl, n-Hexyl und Isohexyl ausgewählt ist; eine Acetylgruppe, eine Methylcyclopropangruppe, eine Aralkylgruppe oder eine Arylgruppe,
wobei die Arylgruppe unter einer Biphenyl-, Cinnamyl- oder Naphthylgruppe ausgewählt ist, die ein- oder zweifach substituiert sein kann mit einem Halogenatom, einer Gruppe CF₃, einer Alkylgruppe mit 1 bis 6 Kohlenstoffatomen, einer Alkoxygruppe mit 1 bis 6 Kohlenstoffatomen, einer Nitrofunktion, einer Polyethergruppe, einer Arylgruppe, einer Benzoylgruppe, einer Alkylestergruppe, einer Carbonsäurefunktion, einer Hydroxygruppe, die gegebenenfalls mit einer Acetylgruppe oder Benzoylgruppe geschützt ist, oder einer Aminofunktion, die gegebenenfalls mit einer Acetylgruppe oder Benzoylgruppe geschützt oder gegebenenfalls mit mindestens einer Alkylgruppe mit 1 bis 6 Kohlenstoffatomen substituiert ist, und
wobei die Aralkylgruppe unter Phenethyl oder Naphth-2-ylmethyl ausgewählt ist, die unsubstituiert vorliegen oder mit einer oder mehr Gruppen substituiert sind, die ausgewählt sind unter einem Halogenatom, einer Gruppe CF₃, einer Alkylgruppe mit 1 bis 6 Kohlenstoffatomen, einer Alkoxygruppe mit 1 bis 6 Kohlenstoffatomen, einer Hydroxygruppe oder einer Aminofunktion, die ungeschützt oder unsubstituiert oder gegebenenfalls mit mindestens einer Alkylgruppe mit 1 bis 6 Kohlenstoffatomen substituiert ist, oder einer Carboxyfunktion, und
wobei R₂. R₄ und R₅ die oben angegebenen Bedeutungen aufweisen;
indem die Verbindung 13 in Gegenwart von Silberoxid mit einem Alkylhalid umgesetzt wird;
n) Herstellung der Verbindung (I): 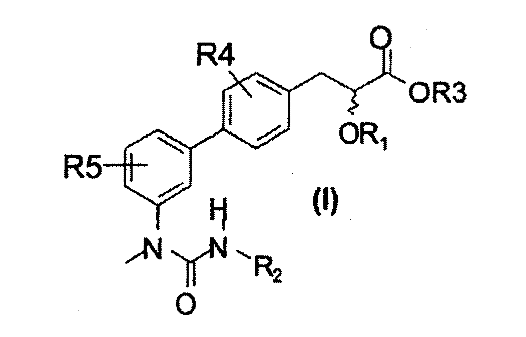
wobei R₃ ein Wasserstoffatom bedeutet,
durch Verseifung der Verbindung 8 in Gegenwart einer Base.

## Revendications

1. Composé de formule (I) : 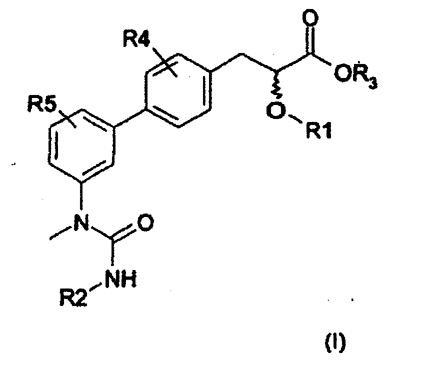 dans laquelle :
- R1 représente un groupe alkyle ayant de 1 à 6 atomes de carbone choisi parmi les radicaux méthyle, éthyle, n-propyle, isopropyle, isobutyle, tert-butyle, n-pentyle, isopentyle, n-hexyle, et isohexyle ; un radical cyclopropyle, un radical cyclohexyle, un radical éthylényle, un radical allyle, un radical propényle, un radial butényle, un radical pentényle ou un radical hexényle, un groupe acétyle, un groupe méthylcyclopropane, un groupe aralkyle ou un groupe aryle, le groupe aryle étant choisi parmi un radical diphényle, cinnamyle ou naphtyle qui peut être mono- ou disubstitué par un atome d'halogène, un radical CF₃, un radical alkyle ayant de 1 à 6 atomes de carbone, un radical alcoxy ayant de 1 à 6 atomes de carbone, un groupe fonctionnel nitro, un radical polyéther, un radical aryle, un radical benzoyle, un groupe ester alkylique, un acide carboxylique, un radical hydroxyle éventuellement protégé par un groupe acétyle ou benzoyle, ou un groupe fonctionnel amino éventuellement protégé par un groupe acétyle ou benzoyle ou éventuellement substitué par au moins un groupe alkyle ayant de 1 à 6 atomes de carbone, et le groupe aralkyle étant choisi parmi un radical phénéthyle ou naphth-2-ylméthyle qui est non substitué ou substitué par un ou plusieurs radicaux choisis parmi un atome d'halogène, un radical CF₃, un radical alkyle ayant de 1 à 6 atomes de carbone, un radical alcoxy ayant de 1 à 6 atomes de carbone, un radical hydroxyle ou un groupe fonctionnel amino qui est non protégé ou non substitué ou éventuellement substitué par au moins un groupe alkyle ayant de 1 à 6 atomes de carbone, ou un groupe fonctionnel carboxyle ;
- R2 représente un groupe alkyle ayant 3 à 8 atomes de carbone, ou un radical cyclopropyle, un radical cyclopentyle, un radical cyclohexyle, un radical allyle, un radical propényle, un radical butényle, un radical pentényle, un radical hexényle, un radical heptényle ou un radical octényle ;
- R3 est un atome d'hydrogène ;
- R4 et R5 représentent un atome d'hydrogène ;
et les isomères possibles, optiques et/ou géométriques, purs ou sous forme de mélange, dans toutes les proportions, dudit composé de formule (I) et les formes tautomères possibles, et également les sels dudit composé de formule (I).

2. Composé selon la revendication 1, **caractérisé en ce qu'**il est obtenu sous forme d'un sel de métal alcalin ou d'un sel de métal alcalinoterreux ou d'un sel d'amine organique.

3. Composé selon la revendication 2, **caractérisé en ce qu'**il est obtenu sous forme d'un sel de sodium.

4. Composé selon la revendication 2, **caractérisé en ce qu'**il est obtenu sous forme d'un sel d'acide aminé.

5. Composé selon la revendication 4, **caractérisé en ce qu'**il est obtenu sous forme d'un sel d'arginine ou d'un sel de lysine.

6. Composé selon l'une des revendications 1 à 5, **caractérisé en ce que** les radicaux alkyle ayant de 1 à 6 atomes de carbone sont choisis parmi les radicaux méthyle, éthyle, n-propyle, isopropyle, n-butyle, isobutyle, tert-butyle, n-pentyle, isopentyle, n-hexyle, et isohexyle.

7. Composé selon l'une des revendications 1 à 5, **caractérisé en ce que** les radicaux alkyle ayant de 3 à 8 atomes de carbone sont choisis parmi les radicaux n-propyle, isopropyle, n-butyle, isobutyle, tert-butyle, n-pentyle, isopentyle, n-hexyle, isohexyle, n-heptyle, isoheptyle, n-octyle et isooctyle.

8. Composé selon l'une des revendications 1 à 5, **caractérisé en ce que** l'atome d'halogène est un atome de fluor, de brome ou de chlore.

9. Composé selon l'une des revendications 1 à 5, **caractérisé en ce que** le radical alcoxy est un radical méthoxy, éthoxy, isopropyloxy, tert-butoxy, hexyloxy, benzyloxy ou phénoxy qui peut éventuellement être substitué par un radical alkyle ayant de 1 à 6 atomes de carbone.

10. Composé selon l'une des revendications 1 à 5, **caractérisé en ce que** le radical polyéther est un radical ayant de 1 à 6 atomes de carbone qui est interrompu par au moins un atome d'oxygène, tel que les radicaux méthoxyméthoxy, méthoxyméthylène, éthoxyméthoxy, éthoxyméthylène ou méthoxyéthoxyméthoxy.

11. Composé selon l'une des revendications 1 à 5, **caractérisé en ce que** le radical ester alkylique est un groupe fonctionnel carboxylate substitué par un radical alkyle ayant de 1 à 6 atomes de carbone.

12. Composé selon la revendication 1, **caractérisé en ce qu'**il représente, seul ou sous forme d'un mélange, un élément de l'ensemble formé par :
1. l'acide 2(S)-éthoxy-3-[3'-(3-heptyl-1-méthyluréido)-biphényl-4-yl]propanoïque,
2. l'acide 2(S)-éthoxy-3-[3'-(1-méthyl-3-pentyluréido)-biphényl-4-yl]propanoïque,
3. l'acide 2(S)-cyclopropylméthoxy-3-[3'-(3-heptyl-1-méthyluréido)biphényl-4-yl]propanoique,
4. l'acide 2(S)-propyloxy-3-[3'-(3-heptyl-1-méthyluréido)-biphényl-4-yl]propanoique,
6. l'acide 2(S)-allyloxy-3-[3'-(3-heptyl-1-méthyluréido)-biphényl-4-yl]propanoique,
7. l'acide 2(S)-cyclopropylméthoxy-3-[3'-(1-méthyl-3-pentyluréido)biphényl-4-yl]propanoïque,
8. l'acide 3-[3'-(1-méthyl-3-pentyluréido)biphényl-4-yl]-2(S)-propoxypropanoïque,
9. l'acide 2(S)-allyloxy-3-[3'-(1-méthyl-3-pentyluréido)-biphényl-4-yl]propanoïque,
11. l'acide 2(S)-méthoxy-3-[3'-(1-méthyl-3-pentyluréido)-biphényl-4-yl]propanoique,
12. l'acide 3-[3'-(3-heptyl-1-méthyluréido)-biphênyl-4-yl]-2(S)-méthoxypropanoïque,
13. l'acide 2(S)-éthoxy-3-[3'-(1-méthyl-3-propyluréido)biphényl-4-yl]propanoïque,
14. l'acide 3-[3'-(3-cyclopropylméthyl-1-méthyluréido)biphényl-4-yl]-2(S)-éthoxypropanoïque,
15. l'acide 3-[3'-(3-cyclopentylméthyl-1-mêthyluréido)biphényl-4-yl]-2(S)-éthoxypropanoïque,
21. l'acide 2(S)-cyclopropylméthoxy-3-[3'-(3-cyclopropylméthyl-1-méthyluréido)biphényl-4-yl]propanoïque,
22. l'acide 3-{3'-[3-(2-cyclohexyléthyl)-1-méthyluréido]biphényl-4-yl}-2(S)-éthoxypropanoique,
23. l'acide 2(R)-éthoxy-3-[3'-(1-méthyl-3-pentyluréido)biphényl-4-yl]propanoïque,
24. l'acide 2(R)-éthoxy-3-[3'-(3-heptyl-1-méthyluréido)biphényl-4-yl]propanoique,
25. l'acide 2-éthoxy-3-[3'-(3-heptyl-1-méthyluréido)biphényl-4-yl]propanoïque,
26. l'acide 2(R)-allyloxy-3-[3'-(3-heptyl-1-méthyluréido)biphényl-4-yl]propanoique,
27. l'acide 3-[3'-(3-allyl-1-méthyluréido)biphényl-4-yl]-2(S)-éthoxypropanoïque.

13. Composé selon l'une des revendications 1 à 5, **caractérisé en ce qu'**il présente au moins une des caractéristiques suivantes :
- R1 est choisi parmi un radical alkyle choisi parmi les radicaux méthyle, éthyle, n-propyle, isopropyle, isobutyle ou tert-butyle, un radical cyclopropyle, ou un groupe méthylcyclopropane,
- R2 représente un radical alkyle choisi parmi les radicaux n-pentyle, isopentyle, n-hexyle, isohexyle, n-heptyle ou isoheptyle, ou un radical cyclopentyle ou cyclohexyle,
- R3 représente un atome d'hydrogène,
- R4 et R5 désignent un atome d'hydrogène.

14. Composition cosmétique, **caractérisée en ce qu'**elle comprend, dans un excipient physiologiquement acceptable, au moins un des composés tels que définis dans l'une quelconque des revendications 1 à 13.

15. Composition selon la revendication 14, **caractérisée en ce que** la concentration du composé ou des composés selon l'une des revendications 1 à 13 est comprise entre 0,001% et 3% en poids, par rapport au poids total de la composition.

16. Utilisation cosmétique d'une composition telle que définie dans l'une des revendications 14 et 15 pour l'hygiène corporelle ou capillaire.

17. Composés selon l'une quelconque des revendications 1 à 13, en tant que médicament.

18. Utilisation d'un composé selon l'une quelconque des revendications 1 à 13 dans la fabrication d'une composition destinée à la régulation et/ou à la restauration du métabolisme des lipides cutanés.

19. Utilisation d'un ou plusieurs composés selon l'une quelconque des revendications 1 à 13 dans la fabrication d'une composition pharmaceutique destinée au traitement :
1) des affections dermatologiques liées à un trouble de la kératinisation portant sur la différentiation et la prolifération,
2) d'autres types de troubles de la kératinisation,
3) d'autres affections dermatologiques avec une composante immuno-allergique inflammatoire, avec ou sans trouble de la prolifération cellulaire,
4) de toutes les proliférations cutanées ou épidermiques, bénignes ou malignes, d'origine virale ou non, des proliférations qui peuvent être induites par un rayonnement ultraviolet,
5) d'autres troubles dermatologiques,
6) des affections dermatologiques ou générales avec une composante immunologique,
7) des troubles cutanés dus à une exposition au rayonnement UV, et également afin de réparer ou de combattre le vieillissement cutané, photoinduit ou chronologique ou afin de réduire les kératoses actiniques et les pigmentations ou toute pathologie associée au vieillissement chronologique ou actinique,
8) des troubles de la fonction sébacée,
9) ou à la prévention des troubles de la cicatrisation ou la prévention ou la réparation des vergetures,
10) des troubles de la pigmentation,
11) des affections du métabolisme des lipides,
12) des affections inflammatoires,
13) ou à la prévention des affections cancéreuses ou précancéreuses,
14) ou à la prévention de l'alopécie de différentes origines,
15) des troubles du système immunitaire,
16) des affections du système cardiovasculaire.

20. Utilisation selon la revendication 19 **caractérisée en ce que :**
1) les affections dermatologiques liées à un trouble de la kératinisation portant sur la différentiation et la prolifération sont choisies parmi l'acné vulgaire, l'acné comédonienne ou polymorphe, l'acné rosacée, l'acné nodulo-kystique, l'acné conglobata, l'acné sénile, ou l'acné solaire, médicamenteuse ou professionnelle,
2) les autres types de troubles de la kératinisation sont choisis parmi les ichthyoses, les affections ichthyosiformes, la maladie de Darier, la kératodermie palmo-plantaire, les affections de type leucoplasie et leucoplasiformes ou le lichen cutané ou muqueux (oral),
3) les autres affections dermatologiques avec une composante immuno-allergique inflammatoire, avec ou sans trouble de la prolifération cellulaire, sont choisies parmi toutes les formes de psoriasis, cutané, muqueux ou unguéal, et même le rhumatisme psoriasique, ou alternativement l'eczéma, ou l'atopie respiratoire ou alternativement l'hypertrophie gingivale,
4) les proliférations, bénignes ou malignes, d'origine virale ou non sont choisies parmi les verrues communes, les verrues planes et l'épidermodysplasie verruciforme, les papillomatoses florides ou orales, le lymphome T, l'épithélioma basocellulaire et spinocellulaire, ou les kératoacanthomes,
5) les autres troubles dermatologiques sont choisis parmi les dermatoses auto-immunes, le lupus érythémateux, les maladies auto-immunes bulleuses et les maladies du collagène, ou la sclérodermie,
7) les troubles cutanés dus à une exposition au rayonnement UV, et également afin de réparer ou combattre le vieillissement de la peau, sont la xérose,
8) les troubles de la fonction sébacée sont choisis parmi l'hyperséborrhée de l'acné ou la séborrhée simple,
10) les troubles de la pigmentation sont choisis parmi l'hyperpigmentation, le mélasma, l'hypopigmentation ou le vitiligo,
11) les affections du métabolisme des lipides sont choisies parmi l'obésité, l'hyperlipidémie ou le diabète non insulino-dépendant,
12) les affections inflammatoires sont l'arthrite,
14) la prévention de l'alopécie de différentes origines est l'alopécie due à la chimiothérapie ou aux rayonnements,
15) les troubles du système immunitaire sont choisis parmi l'asthme, le diabète sucré de type 1, la sclérose en plaques ou d'autres dysfonctionnements sélectifs du système immunitaire,
16) l'affection du système cardiovasculaire est l'artériosclérose ou l'hypertension.

21. Composition pharmaceutique, **caractérisée en ce qu'**elle comprend, dans un excipient physiologiquement acceptable, au moins un des composés tels que définis dans l'une quelconque des revendications 1 à 13.

22. Composition selon la revendication 21, **caractérisée en ce que** la concentration du ou des composés selon l'une des revendications 1 à 13 est comprise entre 0,001% et 10% en poids, par rapport au poids total de la composition.

23. Composition selon la revendication 22, **caractérisée en ce que** la concentration du ou des composés selon l'une des revendications 1 à 13 est comprise entre 0,01% et 1% en poids, par rapport au poids total de la composition.

24. Procédé de synthèse des composés de formule (I) ou des isomères possibles, optiques et/ou géométriques, purs ou sous forme de mélange, dans toutes les proportions, desdits composés de formule (I), des formes tautomères possibles, ou des sels desdits composés de formule (I), **caractérisé en ce qu'**il comprend les étapes suivantes :
a) Préparation du composé de formule 1 : 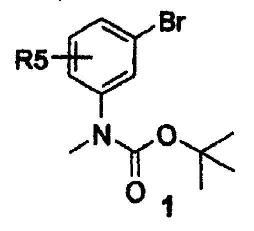 où R5 représente un atome d'hydrogène,
à partir de la 3-bromoaniline, en protégeant l'amine avec du dicarbonate de di(tert-butyle) et ensuite en effectuant une méthylation en présence d'hydrure de sodium ;
b) Préparation du composé 2 : 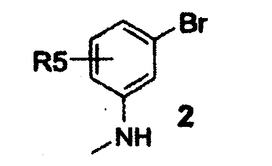 R5 étant tel que défini ci-dessus,
en traitant le composé 1 avec un acide ;
c) Préparation du composé 3 : 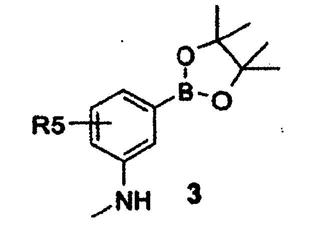 R5 étant tel que défini ci-dessus,
par réaction du composé 2 avec du pinacolborane en présence d'un catalyseur ;
d) Préparation du composé 5 : 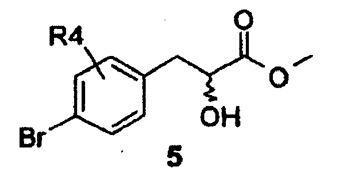 R4 représentant un atome d'hydrogène,
en préparant l'époxyde 4 : 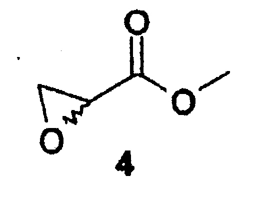 avec un cuprate d'aryle ;
e) Préparation du composé 6 : 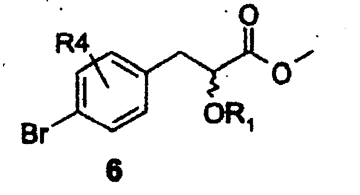 R1 représentant un groupe alkyle ayant de 1 à 6 atomes de carbone choisi parmi les radicaux méthyle, éthyle, n-propyle, isopropyle, isobutyle, tert-butyle, n-pentyle, isopentyle, n-hexyle, et isohexyle ; un radical cyclopropyle, un radical cyclohexyle, un radical éthylényle, un radical allyle, un radical propényle, un radical butényle, un radical pentényle ou un radical hexényle, un groupe acétyle, un groupe méthylcyclopropane, un groupe aralkyle ou un groupe aryle, le groupe aryle étant choisi parmi un radical biphényle, cinnamyle ou naphtyle qui peut être mono- ou disubstitué par un atome d'halogène, un radical CF₃, un radical alkyle ayant de 1 à 6 atomes de carbone, un radical alcoxy ayant de 1 à 6 atomes de carbone, un groupe fonctionnel nitro, un radical polyéther, un radical aryle, un radical benzoyle, un groupe ester alkylique, un acide carboxylique, un radical hydroxyle éventuellement protégé par un groupe acétyle ou benzoyle, ou un groupe fonctionnel amino éventuellement protégé par un groupe acétyle ou benzoyle ou éventuellement substitué par au moins un groupe alkyle ayant de 1 à 6 atomes de carbone, et
le groupe aralkyle étant choisi parmi un radical phénéthyle ou naphth-2-ylméthyle qui est non substitué ou substitué par un ou plusieurs radicaux choisis parmi un atome d'halogène, un radical CF₃, un radical alkyle ayant de 1 à 6 atomes de carbone, un radical alcoxy ayant de 1 à 6 atomes de carbone, un radical hydroxyle ou un groupe fonctionnel amino qui est non protégé ou non substitué ou éventuellement substitué par au moins un groupe alkyle ayant de 1 à 6 carbone atomes, ou un groupe fonctionnel carboxyle,
et R4 étant tel que défini ci-dessus ;
en faisant réagir le composé 5 avec un halogénure d'alkyle en présence d'oxyde d'argent ;
f) Préparation du composé 7 : 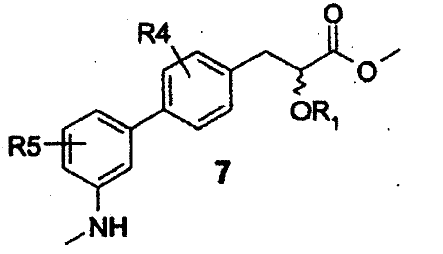 R1, R4 et R5 étant tels que définis ci-dessus,
par couplage du composé 6 et du composé 3 selon une réaction de type Suzuki en présence de tétrakis(triphénylphosphine)palladium ;
g) Préparation du composé 8 : 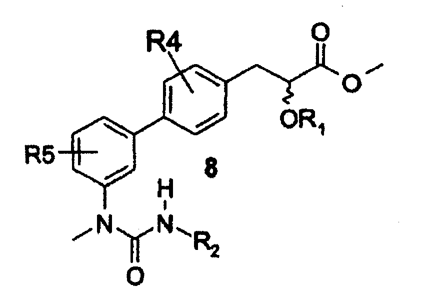 R2 représentant un groupe alkyle ayant de 3 à 8 atomes de carbone, ou un radical cyclopropyle, un radical cyclopentyle, un radical cyclohexyle, un radical allyle, un radical propényle, un radical butényle, un radical pentényle, un radical hexényle, un radical heptényle ou un radical octényle,
et R1, R4 et R5 étant tels que définis ci-dessus,
par réaction entre le composé 7 et un isocyanate d'alkyle ;
h) Préparation du composé (I) : 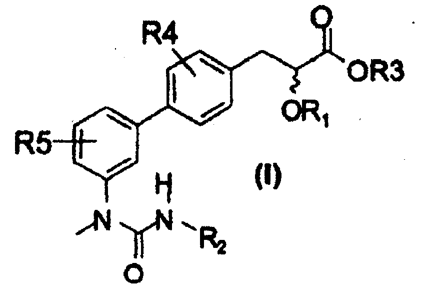
R3 étant un atome d'hydrogène,
par saponification du composé 8 en présence d'une base.

25. Procédé de synthèse des composés de formule (I) ou des isomères possibles, optiques et/ou géométriques, purs ou sous forme de mélange, dans toutes les proportions, desdits composés de formule (I), des formes tautomères possibles, ou des sels desdits composés de formule (I), **caractérisé en ce qu'**il comprend les étapes suivantes :
a) Préparation du composé de formule 1 : 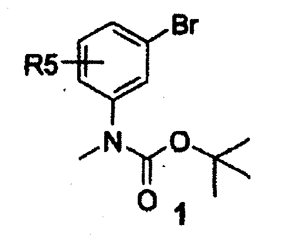 où R5 représente un atome d'hydrogène,
à partir de la 3-bromoaniline, en protégeant l'amine avec du dicarbonate de di(tert-butyle) et ensuite en effectuant une méthylation en présence d'hydrure de sodium ;
b) Préparation du composé 2 : 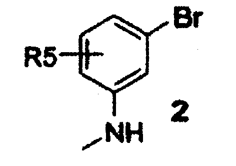 R5 étant tel que défini ci-dessus,
en traitant le composé 1 avec un acide ;
c) Préparation du composé 3 : 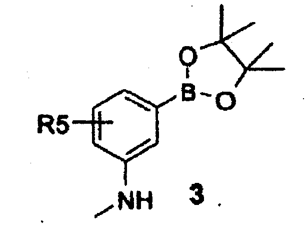 R5 étant tel que défini ci-dessus ;
par réaction du composé 2 avec du pinacolborane en présence d'un catalyseur ;
d) Préparation du composé 5 : 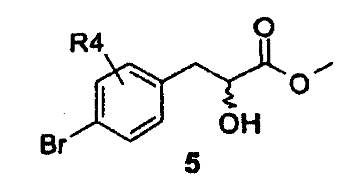 R4 représentant un atome d'hydrogène,
en préparant l'époxyde 4 : 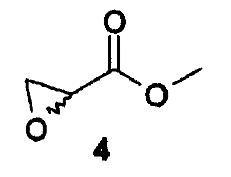 avec un halogénure d'aryle en présence de tert-butyllithium et de cyanure de cuivre ;
i) Préparation du composé 10 : 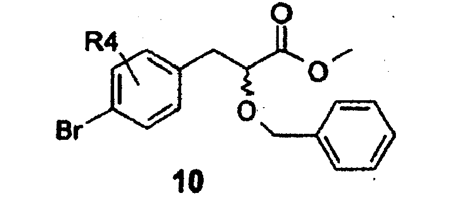 R4 étant tel que défini ci-dessus,
en traitant le composé 5 avec un halogénure de benzyle en présence d'oxyde d'argent ;
j) Préparation du composé 11 : 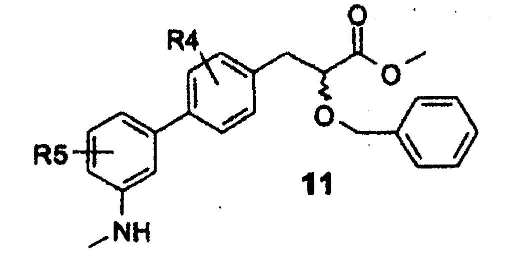 R4 et R5 étant tels que définis ci-dessus,
en couplant le composé 10 avec le composé 3 par une réaction de type de Suzuki en utilisant un catalyseur de palladium ;
k) Préparation du composé 12, dans lequel R2, R4 et R5 sont tels que définis dans la formule (I), 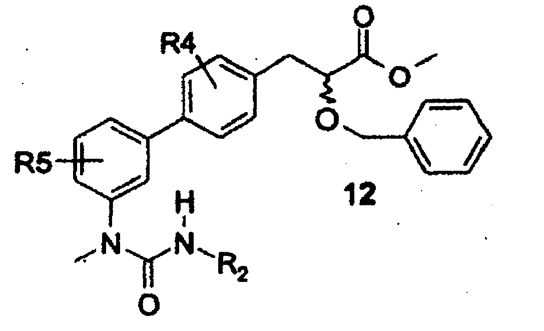 R2 représentant un groupe alkyle ayant de 3 à 8 atomes de carbone, ou un radical cyclopropyle, un radical cyclopentyle, un radical cyclohexyle, un radical allyle, un radical propényle, un radical butényle, un radical pentényle, un radical hexényle, un radical heptényle ou un radical octényle,
et R4 et R5 étant tels que définis ci-dessus,
par réaction entre le composé 11 et un isocyanate d'alkyle ;
l) Préparation de l'alcool 13 : 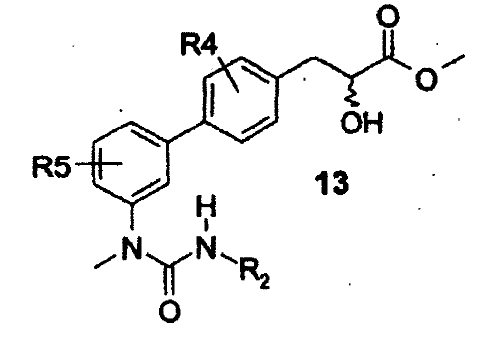 R2, R4 et R5 étant tels que définis ci-dessus,
par hydrogénolyse du composé 12 avec de l'hydrogène en présence de palladium sur charbon ;
m) Préparation du composé 8, dans lequel R1, R2, R4 et R5 sont tels que définis dans la formule (I), 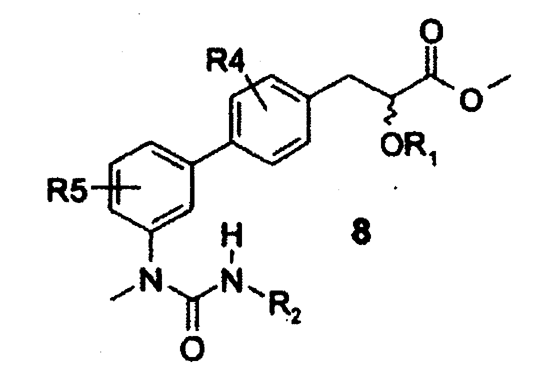 R1 représentant un groupe alkyle ayant de 1 à 6 atomes de carbone choisi parmi les radicaux méthyle, éthyle, n-propyle, isopropyle, isobutyle, tert-butyle, n-pentyle, isopentyle, n-hexyle, et isohexyle ; un groupe acétyle, un groupe méthylcyclopropane, un groupe aralkyle ou un groupe aryle, le groupe aryle étant choisi parmi un radical biphényle, cinnamyle ou naphtyle qui peut être mono- ou disubstitué par un atome d'halogène, un radical CF₃, un radical alkyle ayant de 1 à 6 atomes de carbone, un radical alcoxy ayant de 1 à 6 atomes de carbone, un groupe fonctionnel nitro, un radical polyéther, un radical aryle, un radical benzoyle, un groupe ester alkylique, un acide carboxylique, un radical hydroxyle éventuellement protégé par un groupe acétyle ou benzoyle, ou un groupe fonctionnel amino éventuellement protégé par un groupe acétyle ou benzoyle ou éventuellement substitué par au moins un groupe alkyle ayant de 1 à 6 atomes de carbone, et
le groupe aralkyle étant choisi parmi un radical phénéthyle ou naphth-2-ylméthyle qui est non substitué ou substitué par un ou plusieurs radicaux choisis parmi un atome d'halogène, un radical CF₃, un radical alkyle ayant de 1 à 6 atomes de carbone, un radical alcoxy ayant de 1 à 6 atomes de carbone, un radical hydroxyle ou un groupe fonctionnel amino qui est non protégé ou non substitué ou éventuellement substitué par au moins un groupe alkyle ayant de 1 à 6 atomes de carbone, ou un groupe fonctionnel carboxyle ;
et R2, R4 et R5 étant tels que définis ci-dessus ;
en faisant réagir le composé 13 avec un halogénure d'alkyle en présence d'oxyde d'argent ;
n) Préparation de composé (I) : 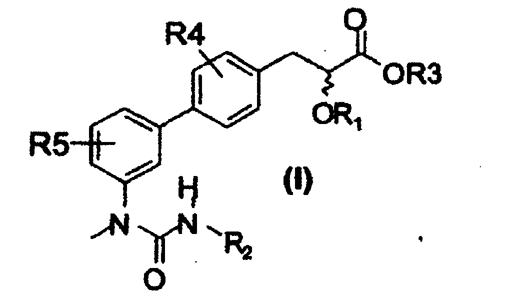 R3 étant un atome d'hydrogène,
par saponification du composé 8 en présence d'une base.
